# EUROPEAN PATENT APPLICATION

(11) **EP 4 675 554 A1**
(43) Date of publication of application: **07.01.2026**
(21) Application number: 25187642.1
(22) Date of filing: 04.07.2025
(51) Int. Cl.: G06T 7/00, A61B 6/03, A61B 6/12, A61B 6/46, A61B 6/50

(54) **MEDICAL IMAGE PROCESSING APPARATUS, MEDICAL IMAGE PROCESSING METHOD AND PROGRAM**

(30) Priority: 04.07.2024 JP 2024108064
(71) Applicant: Canon Medical Systems Corporation, Tochigi 324-0036 (JP)
(72) Inventor: HIGAMI, Hirooki, Gifu, 500-8384 (JP); AOYAMA, Gakuto, Otawara-shi, 324-0036 (JP)
(74) Representative: Marks & Clerk LLP

(57) **Abstract**

According to one embodiment, a medical image processing apparatus includes a first specifying unit, a second specifying unit, and an evaluation unit. The first specifying unit specifies a position of a region of interest of a biological organ from a three-dimensional medical image including image information regarding a treatment device having a plurality of openings. The second specifying unit specifies positions of the openings from the three-dimensional medical image. The evaluation unit evaluates at least one of the openings based on the position of the region of interest and positions of the openings.

## Description

### FIELD

Embodiments described herein relate generally to a medical image processing apparatus, a medical image processing method and a program

### BACKGROUND

The aortic valve of a heart has three leaflets that open and close, and the junctions (commissures) between the respective leaflets are approximately 120° separated. The aortic valve is located between the left ventricle of the heart and the aorta to allow blood to flow from the heart to the aorta by opening and closing the leaflets as the heart moves. In the aortic valve, an aortic valve stenosis may occur in which opening of the leaflets is limited and narrowed due to various causes.

Transcatheter aortic valve implantation (TAVI) is known as a treatment for the aortic valve stenosis. In the TAVI, a prosthetic valve (treatment device) that replaces the patient's aortic valve is carried along a blood vessel using a catheter and retained roughly in a position corresponding to the aortic valve. Note that the prosthetic valve has a complicated structure in which it includes a substantially cylindrical frame having a mesh-like porous surface and three leaflets that can be opened and closed are held inside the frame. Hereinafter, each hole in the frame of the prosthetic valve will be referred to as a cell.

In addition, as a treatment for a coronary artery disease, there is percutaneous coronary intervention (PCI). The PCI is performed for patients who develop a coronary artery disease after TAVI. In the PCI, a catheter is inserted into the organ of interest (the ostium of the coronary artery) while avoiding the retained prosthetic valve (treatment device), and a stent is guided and into the narrowed area of the coronary artery.

The post-TAVI PCI is more difficult than conventional PCI because of a prosthetic valve on the catheter path to the coronary artery.

Since, furthermore, the prosthetic valve has a complicated structure and the position and shape of the coronary artery vary from patient to patient, the positional relationship between the region of interest (the ostium of the coronary artery) of the biological organ of each patient and each cell of the prosthetic valve (each opening of the treatment device) cannot easily be grasped. This makes it difficult to identify and select an appropriate cell to insert a catheter into the ostium of the coronary artery for each patient.

### SUMMARY

In relation to the embodiments, the following additional notes are disclosed as aspects and selective features of the invention.

(Additional Note 1) A medical image processing apparatus includes: a first specifying unit configured to specify a position of a region of interest of a biological organ from a three-dimensional medical image including image information regarding a treatment device having a plurality of openings; a second specifying unit configured to specify positions of the openings from the three-dimensional medical image; and an evaluation unit configured to evaluate at least one of the openings based on the position of the region of interest and positions of the openings. For example, the evaluation may indicate, the accessibility of an opening for allowing passage of a catheter (CT) from inside the treatment device, through the opening, to the specified position of the region of interest.

(Additional Note 2) The medical image processing apparatus may further include a display control unit. The evaluation unit may be configured to create evaluation information indicating a result of the evaluation in association with the positions of the openings. The display control unit may be configured to display the evaluation information on a display.

(Additional Note 3) The treatment device may be a prosthetic valve including a mesh-like substantially cylindrical frame including a plurality of cells. The openings may correspond to the cells.

(Additional Note 4) Positions of the cells may be indices corresponding to places of the substantially cylindrical frame in a circumferential direction and places thereof in a height direction.

(Additional Note 5) The evaluation information may be one of information representing the result of the evaluation in a tabular format in association with the places in the circumferential direction and the places in the height direction and information representing the result of the evaluation, which is superimposed on one of a schematic diagram and a picture of the frame, in association with the places in the circumferential direction and the places in the height direction.

(Additional Note 6) The evaluation information may be information representing the result of the evaluation by a display attribute corresponding to the result of the evaluation.

(Additional Note 7) The evaluation information may include a result of evaluating, among the openings, an opening located close to the region of interest relatively higher than other openings.

(Additional Note 8) The evaluation information may represent a result of evaluating, among the openings, a first blocked portion corresponding to a lower part located inside a heart valve of a subject and a second blocked portion located from a commissure of leaflets of the prosthetic valve to the lower part relatively lower than openings different from the first blocked portion and the second blocked portion.

(Additional Note 9) The evaluation information may include a result of evaluating, among the different openings, an upper opening located at an upper part on an opposite side of the lower part relatively higher than intermediate openings different from the upper opening.

(Additional Note 10) The evaluation information may include a result of evaluating, among the intermediate openings, a first adjacent opening adjacent to the first blocked portion relatively lower than first intermediate openings different from the first adjacent opening.

(Additional Note 11) The evaluation information may include a result of evaluating, among the first intermediate openings, a second adjacent opening adjacent to the second blocked portion relatively lower than a second intermediate opening different from the second adjacent opening.

(Additional Note 12) The medical image processing apparatus may further include a first acquisition unit configured to acquire form information of the treatment device. The evaluation unit may be configured to evaluate at least one of the openings based on the form information, the position of the region of interest, and the positions of the openings.

(Additional Note 13) The medical image processing apparatus may further include a second acquisition unit configured to acquire shape information of a catheter for use in treatment of a subject. The evaluation unit may be configured to evaluate at least one of the openings based on the shape information, the position of the region of interest, and the positions of the openings.

(Additional Note 14) The medical image processing apparatus may further include: a third acquisition unit configured to acquire an X-ray image including the treatment device; and a display control unit configured to display the X-ray image on a display. The evaluation unit may be configured to select a position of the evaluated at least one of the openings based on a result of the evaluation. The display control unit may be configured to highlight on the display a position corresponding to the selected position on the X-ray image.

(Additional Note 15) A medical image processing method includes: specifying a position of a region of interest of a biological organ from a three-dimensional medical image including image information regarding a treatment device having a plurality of openings; specifying positions of the openings from the three-dimensional medical image; and evaluating at least one of the openings based on the position of the region of interest and positions of the openings.

(Additional Note 16) A program including computer executable instructions, wherein the instructions, when executed by a processor, cause the processor to perform a method including: specifying a position of a region of interest of a biological organ from a three-dimensional medical image including image information regarding a treatment device having a plurality of openings; specifying positions of the openings from the three-dimensional medical image; and evaluating at least one of the openings based on the position of the region of interest and positions of the openings.

### BRIEF DESCRIPTION OF THE DRAWINGS

FIG. 1 is a block diagram showing an example of a configuration of a medical image processing system including a medical image processing apparatus according to an embodiment.
FIG. 2 is a flowchart illustrating the operation of the system in the embodiment.
FIG. 3 is a top view showing an example of a prosthetic valve in the embodiment.
FIG. 4 is a perspective view showing an example of the prosthetic valve in the embodiment.
FIG. 5 is a schematic view showing an example of another prosthetic valve in the embodiment.
FIG. 6 is a schematic view showing an example of still another prosthetic valve in the embodiment.
FIG. 7 is a schematic view illustrating cell addresses of the prosthetic valve in the embodiment.
FIG. 8 is a schematic view illustrating a prosthetic valve different from that of FIG. 7.
FIG. 9 is a top view illustrating the prosthetic valve different from that of FIG. 7.
FIG. 10 is a top view illustrating the prosthetic valve of FIGS. 8 and 9.
FIG. 11 is a schematic view illustrating a prosthetic valve different from those of FIGS. 7 to 10.
FIG. 12 is a top view illustrating a prosthetic valve different from those of FIGS. 7 to 10.
FIG. 13 is a top view illustrating the prosthetic valve of FIG. 12.
FIG. 14 is a diagram illustrating an example of evaluation information in the embodiment.
FIG. 15 is a schematic view illustrating an operation in the embodiment.
FIG. 16 is a schematic sectional view taken along line XVI-XVI of FIG. 15.
FIG. 17 is a schematic view illustrating an operation in the embodiment.
FIG. 18 is a schematic sectional view taken along line XVIII-XVIII of FIG. 17.
FIG. 19 is a diagram illustrating an example of evaluation information in the embodiment.
FIG. 20 is a diagram illustrating another example of the evaluation information in the embodiment.
FIG. 21 is a diagram illustrating another example of the evaluation information in the embodiment.
FIG. 22 is a diagram illustrating still another example of the evaluation information in the embodiment.
FIG. 23 is a diagram illustrating another example of the evaluation information in the embodiment.
FIG. 24 is a diagram illustrating yet another example of the evaluation information in the embodiment.
FIG. 25 is a diagram illustrating yet another example of the evaluation information in the embodiment.
FIG. 26 is a schematic view illustrating an operation in the embodiment.
FIG. 27 is a schematic view illustrating an operation in the embodiment.
FIG. 28 is a top view illustrating a first modification to the embodiment.
FIG. 29 is a schematic sectional view taken along line XXIX-XXIX of FIG. 28.
FIG. 30 is a schematic view illustrating the first modification to the embodiment.
FIG. 31 is a schematic view illustrating a second modification to the embodiment.

### DETAILED DESCRIPTION

Hereinafter, a medical image processing apparatus, a medical image processing method and a program according to an embodiment will be described with reference to the drawings. Note that the following description is directed to a case where information concerning the guidance of a catheter is generated in PCI after TAVI based on information concerning the form of a retained prosthetic valve and information concerning the relationship in position between the prosthetic valve and the ostium of the coronary artery which are obtained from CT images. However, a treatment target is not limited to the case, but any information or treatment can be utilized as a target as long as the relationship between a treatment device retained in the body and the anatomical structure of a new treatment target other than a target treated by the treatment device is information generated to support an important treatment or its planning. In the following description, components having substantially the same function and configuration are denoted by the same reference symbols, and their duplicate descriptions are given as appropriate and necessary.

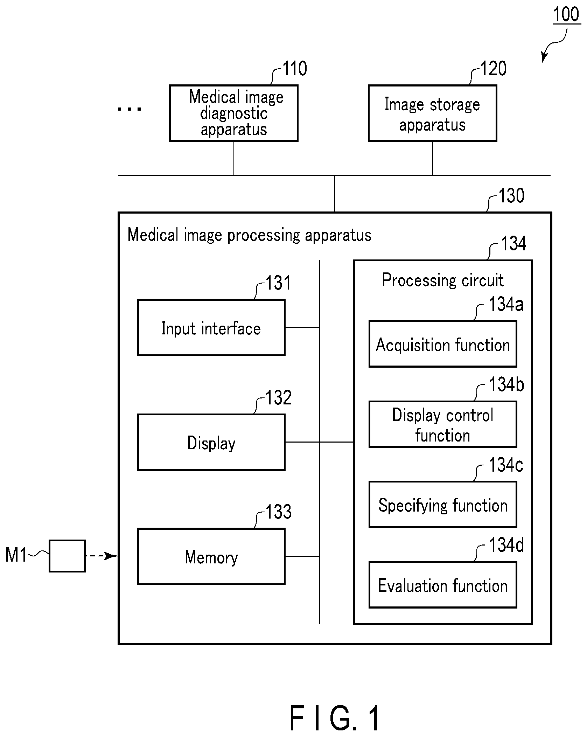

FIG. 1 is a block diagram showing an example of a configuration of a medical image processing system 100 according to an embodiment. The medical image processing system 100 includes a plurality of medical image diagnostic apparatuses 110, an image storage apparatus 120 and a medical image processing apparatus 130. As shown in FIG. 1, the apparatuses 110, 120 and 130 are communicably connected to each other via a network, whether it is wireless or wired. The network is, for example, a local area network (LAN). If security is ensured by a virtual private network (VPN) or the like, the line to be connected is not limited to the LAN. In this case, the network may be a public communication line such as the Internet.

Note that the medical image processing system 100 may be implemented, for example, in the form of a thin client in which a client apparatus used by an operator executes the minimum necessary process and a server apparatus executes most of the processes. At this time, the medical image processing apparatus 130 functions as a server apparatus. If the medical image processing system 100 is implemented in the form of a thin client, the client apparatus (not shown) is connected to the network as, for example, a terminal apparatus including an input interface, a display, a memory, and processing circuitry having a control function, which will be described later. The terminal apparatus may function as a medical image display apparatus, for example.

The medical image diagnostic apparatuses 110 take images for a patient and collect medical image data. The medical image diagnostic apparatuses 110 take, for example, a patient in which a treatment device is retained, and collect medical image data including image information regarding the treatment device. The medical image diagnostic apparatuses 110 transmit the collected medical image data to the image storage apparatus 120 and the medical image processing apparatus 130. For more specific descriptions, it is assumed that the medical image diagnostic apparatuses 110 are X-ray computed tomography (CT) apparatuses. Note that the medical image diagnostic apparatuses 110 are not limited to the X-ray CT apparatuses, but may be any other imaging apparatuses capable of acquiring medical image data, such as an X-ray diagnostic apparatus and a magnetic resonance imaging (MRI) apparatus.

The X-ray CT apparatus collects medical image data on a patient by CT scanning for the patient. CT scanning conditions are attached to the medical image data. The CT scan conditions include, for example, an imaging target region, a scanning method, the number of views, a tube voltage, a tube current, and a patient's position (posture) at the time of CT scanning.

The image storage apparatus 120 stores medical image data collected by the medical image diagnostic apparatuses 110. The image storage apparatus 120 acquires medical image data from the medical image diagnostic apparatuses 110 via the network, and causes the acquired medical image data to be stored in a memory provided inside or outside the apparatus. For example, the image storage apparatus 120 is implemented by a computer apparatus such as a server apparatus.

The medical image processing apparatus 130 acquires medical image data from the medical image diagnostic apparatuses 110 or the image storage apparatus 120 via the network from a communication interface (not shown), and performs a variety of processes using the acquired medical image data. The medical image processing apparatus 130 is implemented by, for example, a computer apparatus such as a workstation. Note that that the medical image diagnostic apparatuses 110, image storage apparatus 120 and medical image processing apparatus 130 can be installed in any location as long as they can be connected via the network. For example, the medical image processing apparatus 130 may be installed in a facility or hospital different from that for the medical image diagnostic apparatuses 110. In addition, the medical image processing apparatus 130 may be mounted on the medical image diagnostic apparatuses 110 such as an X-ray diagnostic apparatus.

As shown in FIG. 1, the medical image processing apparatus 130 includes an input interface 131, a display 132, a memory 133 and processing circuitry 134.

The input interface 131 is implemented by a track ball for inputting various instructions, commands, information, selection and setting from an operator (user) to the main body of the medical information processing apparatus, a switch button, a mouse, a keyboard, a touch pad (or a track pad) for performing an input operation by touching an operation surface, a touch panel display (or a touch screen) in which the display screen and the touch pad are integrated as one unit, and the like. The input interface 131 is connected to the processing circuitry 134 to convert the input operation received from the user into an electrical signal and then output it to the processing circuitry 134. In this case, the input interface 131 may cause a graphical user interface (GUI) to be displayed on the display 132 for the user to input various instructions using physical operating components such as a mouse and keyboard. Note that in the present specification, the input interface 131 is not limited to one including physical operating components. The input interface 131 includes, for example, a circuit for processing an electrical signal that is received in response to an input operation from an external input apparatus provided separately from the apparatus and then output to the processing circuitry 134. In the following descriptions, "user operation of input interface 131" is also referred to as "user operation."

The display 132 includes a display body that displays optional data, an internal circuit that supplies a signal for display to the display body, and a peripheral circuit such as a connector and cable for connecting the display body and the internal circuit. The display 132 displays various items of information under the control of a display control function 134b in the processing circuitry 134. For example, the display 132 displays various images generated by the processing circuitry 134. The display 132 also displays a GUI as the input interface 131. Various optional displays can be used appropriately as the display 132. For example, the display 132 may be a liquid crystal display, an electro-luminescence display, or a plasma display. The display 132 may be a desktop type or may include a tablet terminal capable of wireless communication with the medical image processing apparatus 130.

The memory 133 includes a memory for recording electrical information, such as a read only memory (ROM), a random access memory (RAM), a hard disk drive (HDD) and an image memory, and a peripheral circuit such as a memory controller and a memory interface which are associated with the memories. The memory 133 stores, for example, programs of the medical image processing apparatus 130 and various items of data such as various tables, data that is being processed, and data that has been processed. Note that the programs cause a computer to function as the medical image processing apparatus 130 that performs a medical image process. The programs may be stored in, for example, a non-transitory computer readable storage medium M1 and distributed, and then read from the storage medium M1 and installed in the memory 133. The programs also include computer executable instructions. The instructions cause a processor to perform a medical image processing method when they are executed by the processor. The medical image processing method includes, for example, specifying the location of a region of interest of a biological organ from a three-dimensional medical image containing image information regarding a treatment device having a plurality of openings, specifying the location of the openings from a three-dimensional medical image, and evaluating at least one of the openings based on the location of the region of interest and the location of the openings.

The processing circuitry 134 reads the programs from the memory 133 based on the instructions input from the user via the input interface 131 to control the medical image processing apparatus 130 in accordance with the programs. For example, the processing circuitry 134 is a processor that fulfills each of the functions of the medical image processing apparatus 130 in accordance with the programs read from the memory 133. The functions include, for example, an acquisition function 134a, a display control function 134b, a specifying function 134c and an evaluation function 134d. Note that the functions may be distributed among a plurality of processors as appropriate. Alternatively, each function or part of each function may be performed by other apparatuses as appropriate.

Next, as the functions of the processing circuitry 134, the acquisition function 134a, display control function 134b, specifying function 134c and evaluation function 134d will be described in the order presented. However, a share of each function described below is expedient and can be changed as needed. This is because even if a process shared by a certain function is performed by another function, the processing circuitry 134 still performs the process. Note that the fact that a share of each function can be changed is true of the following embodiments and modifications.

The acquisition function 134a acquires form information of the treatment device having a plurality of openings. The acquisition function 134a also acquires a three-dimensional medical image including image information regarding a patient's treatment device. For example, the acquisition function 134a may acquire form information of a treatment device from the server of a manufacturer of the treatment device. For example, the acquisition function 134a may acquire the form information of a treatment device from the memory 133 in which the form information is stored. Similarly, the acquisition function 134a may acquire a three-dimensional medical image from either the medical image diagnostic apparatuses 110 or the image storage apparatus 120. For example, the acquisition function 134a may acquire a three-dimensional medical image from the memory 133. The acquisition function 134a is an example of a first acquisition unit.

The display control function 134b causes the display 132 to display data that is being processed by the processing circuitry 134 or data that is obtained as a result of the process of the processing circuitry 134. For example, the display control function 134b may cause the display 132 to display the form information and the three-dimensional medical image acquired by the acquisition function 134a. For example, the display control function 134b may cause the display 132 to display a location specified by the specifying function 134c, a result evaluated by the evaluation function 134d. For example, the display control function 134b may cause the display to display evaluation information created by the evaluation function 134d. The display control function 134b is an example of a display control unit.

The specifying function 134c specifies the location of the region of interest of the biological organ from the three dimensional medical image containing image information regarding the patient's treatment device. For example, the specifying function 134c specifies the location of the ostium of a coronary artery from a three dimensional medical image containing image information regarding the patient's prosthetic valve. Note that the prosthetic valve is retained inside the patient's aorta by TAVI. Here is a supplemental description of an anatomy of the aortic valve. A typical aortic valve of the heart has three leaflets (right coronary cusp, left coronary cusp, and non-coronary cusp) that open and close, and the junctions (commissures) between the respective leaflets are approximately 120° separated. The aortic valve is located between the left ventricle of the heart and the aorta to control blood flow from the heart to the aorta by opening and closing the leaflets as the heart moves. Specifically, blood flows from the left ventricle to the aorta through the aortic valve in the Valsalva sinus. Part of the blood that has passed through the aortic valve flows to the right and left coronary arteries through their respective two coronary artery ostiums in the Valsalva sinus. The prosthetic valve is retained inside the aortic valve that developed an aortic valve stenosis and behaves like a normal aortic valve. Such a prosthetic valve is located in the vicinity of the two coronary artery ostiums. Note that the prosthetic valve is an example of the treatment device. The coronary artery ostiums are an example of an organ region of interest.

The specifying function 134c specifies the locations of the openings of the treatment device from the three-dimensional medical image. The treatment device may be, for example, a prosthetic valve having a mesh-like substantially cylindrical frame composed of a plurality of cells. In this case, the openings in the treatment device correspond to the cells. The locations of the cells may be indices corresponding to the places in the substantially cylindrical frame in its circumferential and height directions. The prosthetic valve is retained inside the patient's aortic valve by TAVI. In the structure of the prosthetic valve, three leaflets that can be opened and closed are held inside the frame. More specifically, like the anatomy of a typical aortic valve, the inner structure of the frame of the prosthetic valve has three leaflets (right coronary cusp, left coronary cusp, and non-coronary cusp) which open and close, and junctions (commissures) which are approximately 120° separated between the respective leaflets. Note that the specifying function 134c may specify the locations of the commissures of the prosthetic valve in addition to the locations of the cells. For example, the specifying function 134c can specify the locations of the commissures of the prosthetic valve based on the location of a marker on the top or bottom of the prosthetic valve. In addition, the openings of the frame may be referred to as struts instead of the cells. The leaflets may be referred to as valve cusps. The specifying function 134c is an example of first and second specifying units.

The evaluation function 134d evaluates at least one of the openings based on the form information of the treatment device, the location of the region of interest of the biological organ and the locations of the openings of the treatment device. The evaluation function 134d may generate evaluation information that represents the results of the evaluation in association with the locations of the openings. For example, the locations of the cells as the openings may be indices corresponding to the places in the substantially cylindrical frame in its circumferential and height directions.

The evaluation information may be either information that indicates the evaluation result in a table format in association with the places in the frame in its circumferential and height directions or information that indicates the evaluation result in association with the places in the frame in its circumferential and height directions by superimposing it on the schematic drawing or picture of the frame. The evaluation information may be information that represents the evaluation result by a display attribute corresponding to the evaluation result. The display attribute may include, for example, at least one of color, symbol, pattern, numerical value and alphabet. The evaluation function 134d is an example of an evaluation unit.

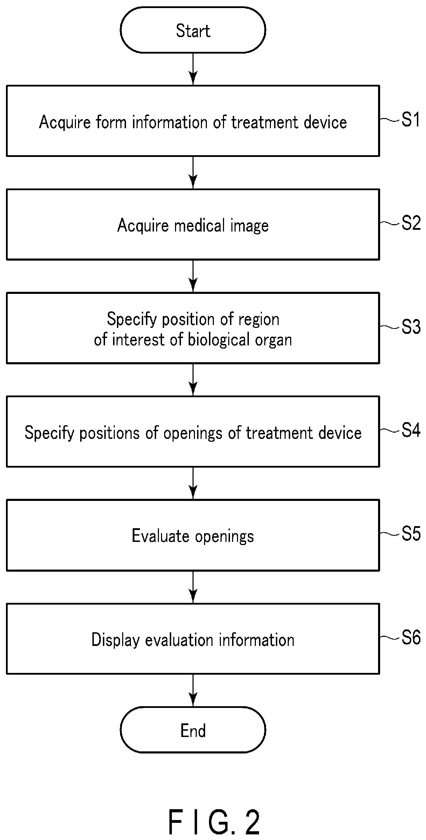

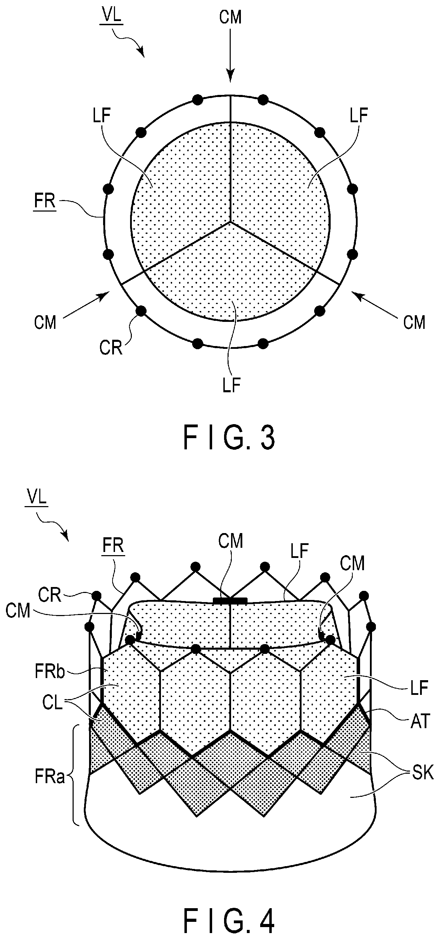

The operation of the medical image processing apparatus configured as described above will be described using the flowchart of FIG. 2 and referring to FIGS. 3 to 27. The following description will be given as an example in which each cell of a prosthetic valve is evaluated based on the location of the ostium of a coronary artery in CT images and the location of the cell in the CT images in the PCI treatment planning. However, the operation of the medical image processing apparatus 130 is not limited to this example.

### (Step S1)

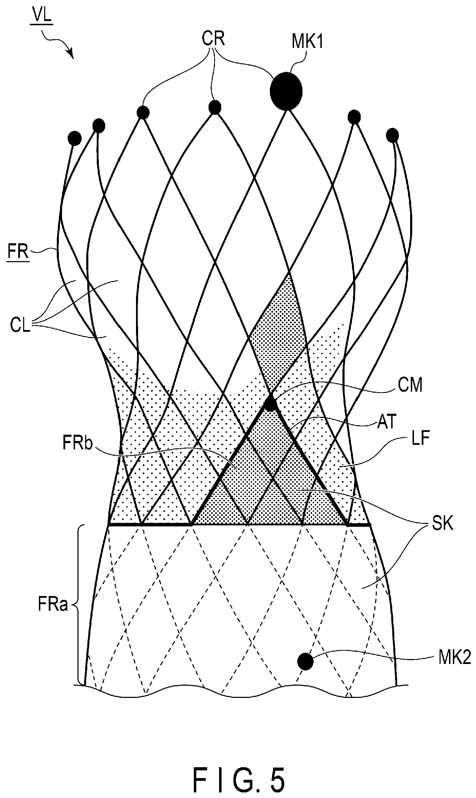

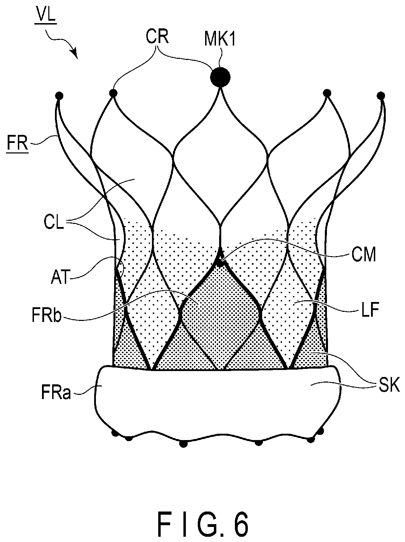

The processing circuitry 134 of the medical image processing apparatus 130 causes the acquisition function 134a to acquire form information of a treatment device having a plurality of openings. For example, the processing circuitry 134 acquires form information of a prosthetic valve used for TAVI. Specifically, main prosthetic valves of TAVI include SAPIEN^{™} 3, Evolut^{™} and Navitor^{™}. FIGS. 3 and 4 are respectively a top view and a perspective view each showing a SAPIEN^{™} prosthetic valve VL. FIG. 5 is a schematic view showing an Evolut^{™} prosthetic valve VL and FIG. 6 is a schematic view showing a Navitor^{™} prosthetic valve VL. Each of the prosthetic valves VL has a mesh-like substantially cylindrical frame FR composed of a plurality of cells CL. The frame FR has coronal tops CR, its lower part is protected by a skirt SK, and its inner lower part holds three leaflets LF that can be opened and closed. Specifically, the frame FR and the leaflets LF are joined at a junction AT indicated by a thick line. The leaflets LF can be opened and closed above the junction AT, and are fixed to the frame FR at the junction AT and a region below the junction. The frame FR may be referred to as a stent. The coronal tops CR may be referred to as crowns. The skirt SK may be referred to as a cuff. The frame FR may also have a marker MK1 at one of the coronal tops CR and a marker MK2 at one of the bottoms. The markers MK1 and MK2 may indicate a location of the frame in its circumferential direction, which corresponds to the location of a commissure CM between the respective leaflets LF. The cells CL include a cell CL through which a catheter can easily pass, a cell CL through which no catheter can pass, and an intermediate cell CL between the two cells. The cell CL through which a catheter can easily pass is located at an upper part of the substantially cylindrical frame FR in which the leaflets LF that open and close does not interfere. The cell CL, through which no catheter can pass (or through which a catheter is very difficult to pass) is located in a region of the substantially cylindrical frame FR which is below the foregoing junction AT. That is, the cell CL through which no catheter can pass (or through which a catheter is very difficult to pass) is located at the lower part FRa of the frame FR located inside the aortic valve of a subject and at a commissure lower part FRb located from the commissure CM of the leaflet LF to the lower part FRa. The intermediate cell CL is located in a part of the substantially cylindrical frame FR, excluding the upper part, lower part FRa and commissure lower part FRb. Though it is possible to cause a catheter to pass through the intermediate cell CL, it is more difficult to cause a catheter to pass therethrough as the catheter comes closer to the lower part FRa and the commissure lower part FRb.

The form information of the prosthetic valve VL includes, for example, identification information (type and size) of the prosthetic valve VL, information regarding the number, arrangement and size of cells CL in the frame FR, and information regarding the positional relationship between a bioprosthetic valve (portions of the leaflets LF and skirt SK) and each of the cells CL. The form information of the treatment device indicates the form of a treatment device having a plurality of openings like the form of a prosthetic valve VL having a plurality of cells CL. Usually, a prosthetic valve VL of a type and size suitable for the anatomy of a patient is selected from among a plurality of types of prosthetic valve having a specified form and is retained. If, therefore, identification information concerning the type and size of the prosthetic valve retained in the patient is acquired, detailed information concerning the form of the prosthetic valve can be acquired from the form information of the prosthetic valve published by each device manufacturer based on the acquired identification information. In addition, the form information concerning the prosthetic valve can also be acquired not only from the web page of each device manufacturer, but also from medical device databases published by a medical device authorized organization in each country, such as Food and Drug Administration (FDA) and Pharmaceuticals and Medical Devices Agency (PMDA). Note that the information concerning the type and size of a treatment device retained in a patient may be acquired from an in-hospital database such as an electronic medical record based on information concerning a treatment history and an operation history, or may be acquired from a referral form or the like from another hospital, or may be acquired from the patient. Alternatively, based on a medical image acquired in step S2 and thereafter, a region of the prosthetic valve depicted in the medical image may be specified and a type of the prosthetic valve may be specified. The prosthetic valve is an artifact and has a different pixel value from those of biological tissues on a medical image. Thus, the region of the prosthetic valve can be specified relatively easily using known region extraction techniques and machine learning techniques. These techniques will be described later.

### (Step S2)

The processing circuitry 134 causes the acquisition function 134a to acquire a three-dimensional medical image (e.g., three-dimensional CT image) containing image information regarding a patient's treatment device (prosthetic valve) from the image storage apparatus 120 or the medical image diagnostic apparatuses 110. Note that the three-dimensional medical image may be of any type as long as the image includes form information of a three-dimensional anatomy of a target biological tissue and the treatment device. For example, the three-dimensional medical image may be a medical image captured by another imaging apparatus such as an ultrasonic image, a magnetic resonance imaging (MRI) image, an X-ray image, a positron emission computed tomography (PET) image and a single photon emission computed tomography (SPECT) image, or may be part of a four-dimensional image obtained by taking a plurality of three-dimensional medical images in time direction. Note that in step S2, the operation may be started by a user, or the image storage apparatus 120 such as a PACS may be monitored so that a new medical image is automatically processed when it is stored in the apparatus 120. Alternatively, the processing circuitry 134 may determine whether the new medical image satisfies a predetermined condition or not, and may process the image if the condition is satisfied. The condition may be any condition under which the state of the image can be determined. For example, a medical image taken under a heart imaging protocol as a condition may be processed, and a medical image enlarged and reconstructed with a reconstruction method as a condition may be processed. In addition, the processing circuitry 134 may perform a process if the combined conditions thereof are satisfied.

Note that step S2 may be executed before step S1.

### (Step S3)

The processing circuitry 134 causes the specifying function 134c to specify the location of a region of interest in the biological organ from the three-dimensional medical image. For example, the processing circuitry 134 specifies, as the location of a region of interest, the location of a region in the biological organ noticed in the acquired CT image. That is, in one embodiment, the processing circuitry 134 specifies coordinate information of each pixel indicated by the coronary artery ostium on the CT image. As a specific process, a region of interest may be specified by operating the input interface 131 to designate the location of the region of interest or it may be specified based on the anatomy depicted on a CT image by known region extraction techniques. The known region extraction techniques include, for example, Otsu's method, region growing method, snakes method, graph-cut method, and mean-shift method, which are based on CT values. Note that in step S3, an optional method for specifying a region of interest from the medical image can be used. For example, a region of interest may be specified using a shape model of a region of interest constructed based on learning data prepared in advance using machine learning techniques (including deep learning). Furthermore, the calculation cost tends to be excessive if the above process is performed on the entire medical image using, for example, the graph-cut method. Therefore, a region of interest may be specified by specifying a region (referred to as a related region hereinafter) which is related to a biological organ of interest and which is larger than the region of interest but smaller than the entire medical image and then applying the above process only to the related region. If, for example, the biological organ of interest is a coronary artery ostium, the related region corresponds to a Valsalva sinus region, a heart region, a region around a mediastinum, and the like. Note that the related region may be set by operating the input interface 131. If the coronary artery ostium is specified as a region of interest, the ostiums of the right and left coronary arteries may be specified as separate regions, or only one of them may be specified. Specifically, in the case of PCI, the location of a coronary artery to be treated is known in advance, and thus only the ostium of the coronary artery that is an access route to the location of the coronary artery may be specified. Note that in step S3, the size of the region of interest is not limited, and the coordinates of only one point (for example, one pixel) may be used as the region of interest.

Note that steps S2 and S3 may be executed before step S1.

### (Step S4)

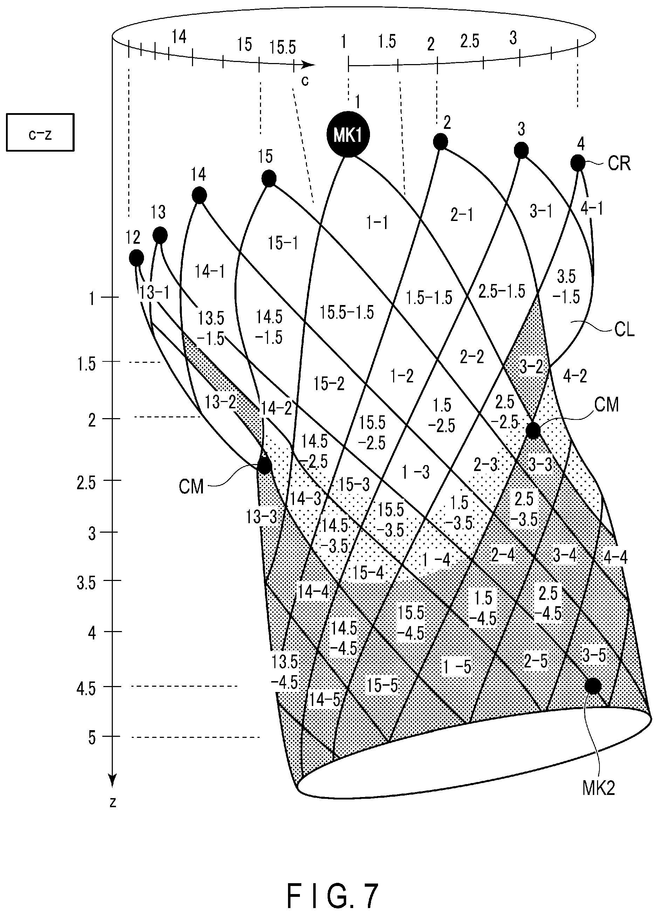

The processing circuitry 134 causes the specifying function 134c to specify the locations of a plurality of openings of a treatment device from a three-dimensional medical image. For example, the processing circuitry 134 acquires coordinate information of each pixel indicating each cell CL of a prosthetic valve VL in the acquired CT image. As a specific process, the locations of the openings may be specified by operating the input interface 131 to designate the locations of the openings or they may be specified based on the structure of the prosthetic valve VL depicted on the CT image by known region extraction techniques. For example, the locations of the openings may be specified using a shape model of a treatment device constructed based on learning data prepared in advance using machine learning techniques (including deep learning). In addition, all regions of the openings in the treatment device may be specified or only some of the regions involved in the treatment may be specified. For example, only cells (openings) located near to the region of interest (the coronary artery ostium in this example) specified in step S3 may be specified. The number of cells to be specified may be at least one if there is one coronary artery in a coronary artery disease. The nearness of the cells to the region of interest may be determined using a predetermined threshold value or may be determined by the user. In addition, a characteristic structure of the prosthetic valve VL may be specified, and information on the positional relationship between each of the openings and the characteristic structure may be specified as positional information. For example, in each cell, information such as a distance from the characteristic structure, an angle and a vector may be specified as positional information. Examples of the characteristic structure may include the structure of the prosthetic valve VL which is distinguishable like the structures of markers MK1 and MK2 and the structure specified by a relative positional relationship among a plurality of structures such as a central position where all the leaflets LF are in contact with each other and a position of the commissures CM. Since the positional relationship between the number and arrangement of cells CL and the above characteristic structure is determined in the prosthetic valve VL, an address is set for each cell in advance based on the characteristic structure to specify a cell CL of a specific address. An example of the addresses of the cells CL will be described below with reference to FIGS. 7 to 13.

FIG. 7 shows an example in which indices corresponding to the places in the substantially cylindrical frame FR in its circumferential and height directions are superimposed on a schematic view of the frame FR and the addresses of the cells CL are assigned. In FIG. 7, the axis of a substantially elliptical shape represents a circumferential direction c, and the vertical axis represents a height direction z. Each of the cells CL is assigned an address that is a combination of the places of the frame FR in its circumferential and height directions.

The places in the circumferential direction c are arranged in ascending order for the first to fifteenth tops CR such that the marker MK1 of the first top CR is set to place 1 and the second top CR is set to place 2. Note that the marker MK1 indicates a reference place in the circumferential direction c and is independent of the commissures CM. In FIG. 7, the marker MK2 at the bottom indicates a position (3) in the circumferential direction corresponding to the position (3-3) of the commissure CM. In FIG. 7, the commissures CM correspond to the third, eighth (not shown) and thirteenth places in the circumferential direction c, and the cells CL in a closed state exist up to place 2 in the height direction as indicated by a dark color.

The places in the height direction z are arranged in ascending order for the cells CL such that the cell CL having a top CR is set to place 1 and the cell CL immediately below the cell CL of place 1 is set to place 2. The places in the height direction z are also arranged in ascending order for the cells CL such that the cell CL which is sandwiched between the cell CL having a top CR and its adjacent cell CL having a top CR and which has no top CR is set to place 1.5 and the cell CL immediately below the cell CL of place 1.5 is set to place 2.5.

Accordingly, in FIG. 7, the address "1-2" of the cell CL indicates the cell of place 1 in the circumferential direction c and place 2 in the height direction z. Note that the address is not limited to a form in which the places in the two directions are connected by a hyphen, but may be a form of coordinates in which the places in the two directions are connected by a comma, and may be expressed in other forms.

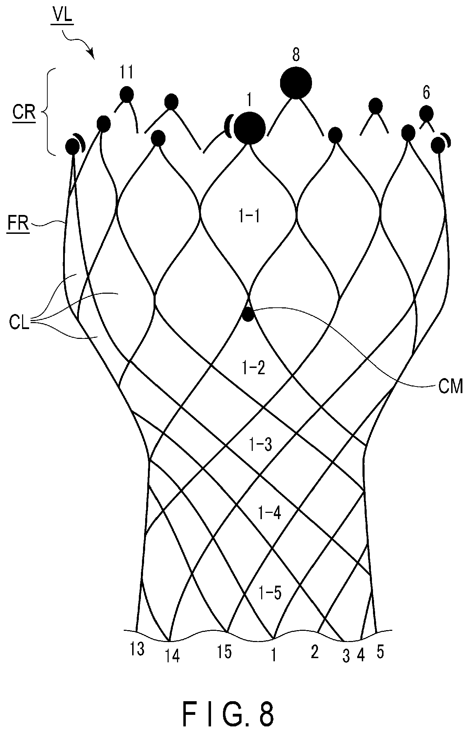

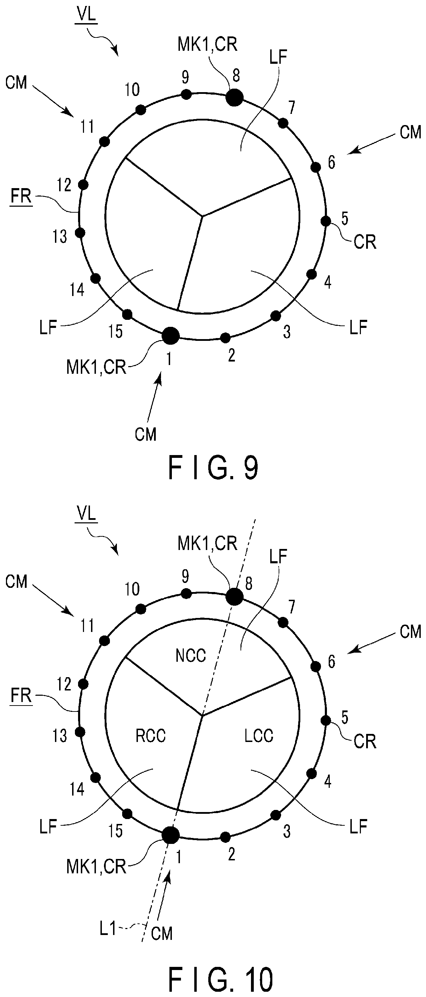

FIGS. 8 and 9 are schematic and top views illustrating an example in which cell addresses similar to those in FIG. 7 are used in a prosthetic valve different from that in FIG. 7. In FIG. 8, the marker MK1 of the first top CR indicates a circumferential position corresponding to the position of one commissure CM. As shown in FIG. 9, the remaining two commissures CM are located at positions corresponding to the sixth and eleventh places in the circumferential direction c. There is also a marker MK1 at the eighth top CR. Here, if the two markers MK1 are connected by a straight line L1 as shown in FIG. 10, there is also a device having a positional relationship in which the left coronary cusp LCC is located alongside six tops CR and the right coronary cusp RCC is located alongside seven tops CR. In such a device, the positional relationship of each valve cusp and commissures CM can be specified based on the number of tops CR. The non-coronary cusp NCC is located at a position where the prosthetic valve is divided by straight line L1. In the three-dimensional medical image of a subject, generally, the ostium of the left coronary artery is located near the left coronary cusp LCC, and the ostium of the right coronary artery is located near the right coronary cusp RCC.

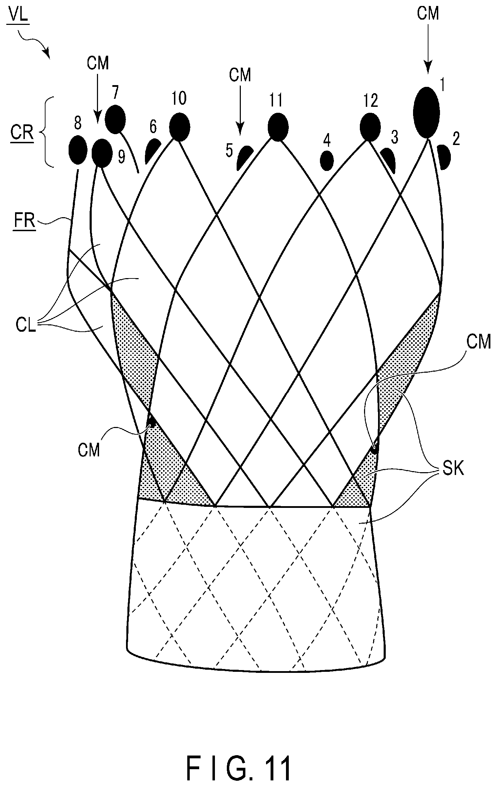

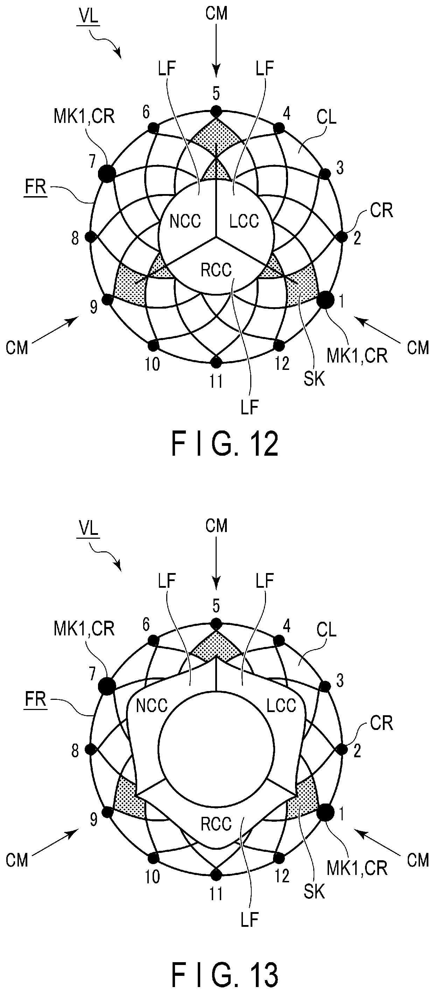

FIGS. 11 and 12 are schematic top views illustrating an example in which cell addresses similar to those in FIG. 7 are used in a prosthetic valve different from those in FIGS. 7 to 10. In FIG. 11, the prosthetic valve VL has 12 tops CR. The marker MK1 of the first top CR indicates a circumferential position corresponding to the position of one commissure CM, and the marker MK1 of the ninth top CR indicates a circumferential position corresponding to the position of another commissure CM. As shown in FIG. 12, the remaining one commissure CM corresponds to the fifth place in the circumferential direction **c.** There is also a marker MK1 at the seventh top CR. If the two markers MK1 are connected by a straight line (not shown), the left coronary cusp LCC is located on one side and the right coronary cusp RCC is located on the other side. The three leaflets LF open and close according to the flow of blood so as to alternate between the closed state shown in FIG. 12 and the open state shown in FIG. 13.

In addition, the position information of all or some of the openings in a treatment device to be specified may be specified based on the same coordinate system as the positional information of a region of interest of a biological organ, and may be specified as information of a positional relationship with the position of the region of interest. That is, the positions of the openings may be specified as absolute positional coordinates in the same coordinate system or as positional information relative to the position of the region of interest of the biological organ. In the latter case, for example, information such as a distance from the target region of the biological organ, an angle and a vector may be specified as positional information.

Note that step S4 may be executed between steps S2 and S3.

In an embodiment, an image of a prosthetic valve is included in both form information of the prosthetic valve acquired in step S1 and a three-dimensional medial image acquired in step S2. The form information of the prosthetic valve in step S1 can be acquired from a web page web page of each device manufacturer. The three-dimensional medical image acquired in step S2 includes image information of the prosthetic valve retained in a patient.

In step S4, the processing circuity 134 may specify locations of openings from locations of openings designated in accordance with operation made to the input interface 131. A user views the prosthetic valves VL in the three-dimensional medical image and respective cells CL, thereby recognizing a cell CL not corresponding to any prosthetic valve VL, as a location of an opening. The user designates a location of an opening on the three-dimensional medical image using the input interface 131 (such as a pointer, etc.). The processing circuitry 134 can specify a location of an opening from coordinate information corresponding to the designated location.

Alternatively, operation to the user interface 131 may be a combination of the image processing (not known region extraction techniques) and processing in lines 16 to 13 of page 21 of the specification. That is, the processing circuitry 134 may be configured such that, in step S4, at the time when a characteristic structure of the prosthetic valve VL is specified through operation to the user interface 131 or image processing, the processing circuitry 134 specifies, as positional information, information of a positional relationship with such a characteristic structure in each opening based on form information of the prosthetic valve. For example, through operation to the input interface 131, the first coronal top CR different from the other coronal tops CR may be specified by designating a location of the marker MK1. The user views the plurality of coronal tops CR in the three-dimensional medical image, thereby recognizing the first coronal top CR different from the other coronal tops CR. The user designates the first coronal top CR with the input interface 131 (such as a pointer). The processing circuit 131 can specify the first coronal top CR as the maker MK1 from coordinate information corresponding to the designated location of the input interface 131. Alternatively, the processing circuitry 134 image-processes the three-dimensional medical image, thereby extracting image regions corresponding to the plurality of coronal tops CR. The processing circuitry 134 may extract an image region which is larger (largest) than a predetermined size among the plurality of image regions of the coronal tops CR and is upwardly protruded, thereby specifying the extracted image region as the marker MK1. In both of the operation to the input interface 131 and the image processing, the processing circuitry 134 can specify a characteristic structure of the prosthetic valve VL. Furthermore, after the characteristic structure is specified, the processing circuitry 134 may specify, as positional information of an opening, information such as a distance from the characteristic structure to the opening, a degree, a vector, etc., in each cell on the basis of the form information of the prosthetic valve, for example. According to an embodiment, the form of the prosthetic valve VL on the three-dimensional medical image can be related to form information of the prosthetic valve acquired in step S1. For example, the markers MK1 and MK2 of the prosthetic valve VL on the three-dimensional medical image can be related/registered to the markers MK1 and MK2 indicated by form information of the prosthetic valve acquired in step S1. After relating/registering them to each other, the orientation and location of the prosthetic valve on the three-dimensional image are specified. Therefore, the location of each opening of the prosthetic valve VL on the three-dimensional image can also be specified.

### (Step S5)

The processing circuitry 134 causes the evaluation function 134d to evaluate at least one of a plurality of openings in a treatment device based on the form information of the treatment device, the position of a region of interest of a biological organ, and the positions of the openings in the treatment device. The processing circuitry 134 also causes the evaluation function 134d to create evaluation information indicating the evaluation result to correspond to the positions of the evaluated openings. Specifically, the processing circuitry 134 calculates an evaluation value according to an evaluation formula predetermined in advance or set by a user, based on the form information of the treatment device and the positional relationship between the region of interest specified in step S3 and the openings specified in step S4. The evaluation value is an example of the evaluation result.

The evaluation value based on the form information of the treatment device is set, for example, based on the distance from the junction of a bioprosthetic valve portion (proximal end of the leaflet LF) in the frame FR of the prosthetic valve VL. The shorter the distance from the junction, the more difficult the cell CL to be accessed by a catheter at the time of PCI. Thus, the evaluation formula is set so that the evaluation value increases with the increasing distance from the junction. The evaluation value may be changed with reference to the height at which the bioprosthetic valve exists. The evaluation values of portions (on the LVOT side and left ventricle side) below the bioprosthetic valve portion may be set to 0 because the portions are usually impossible (or very difficult) to access by a catheter. LVOT stands for left ventricular outflow tract. That is, based on the structure of the treatment device (prosthetic valve), the evaluation value can be calculated for each cell by an evaluation formula set in advance with reference to the degree of influence (accessibility) in another treatment (PCI).

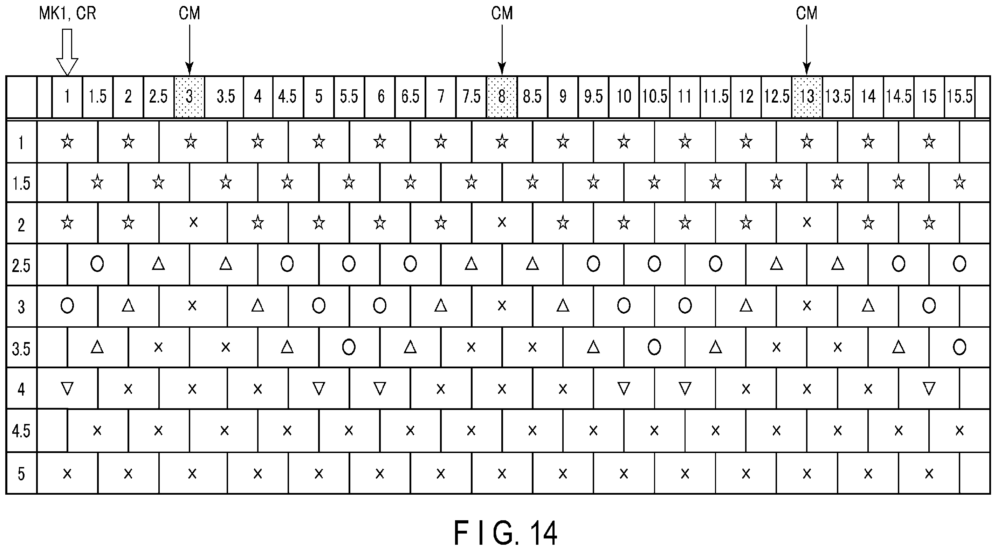

FIG. 14 is a diagram showing an example of evaluation information representing evaluation results of a plurality of cells CL in the prosthetic valve VL shown in FIG. 7. In FIG. 14, the uppermost row represents places 1 to 15.5 in the circumferential direction c. The places 1 to 15.5 in the circumferential direction c may be referred to as column numbers. Note that the marker MK1 of top CR and its corresponding commissures CM are shown for places 1, 3, 8 and 13 in the circumferential direction c for the purpose of easy understanding of the correspondence between FIGS. 7 and 14; however, the marker MK1 and the commissures CM may be omitted because they are not essential. The leftmost column represents places 1 to 5 in the height direction **z.** The places 1 to 5 in the height direction z may be referred to as column numbers. The evaluation values are associated with cells of addresses (locations) corresponding to the places (column numbers) in the circumferential direction c and the places (row numbers) in the height direction **z.**

The evaluation values are represented by five different symbols: cross mark (×), inverted triangle (V), triangle (Δ), circle (∘) and star mark (☆). The evaluation values increase in the order of symbols presented above. That is, the lowest evaluation value is represented by cross mark (×) and the highest evaluation value is represented by star mark (☆).

The cells CL corresponding to the positions of the cross marks (×) are given the lowest evaluation value because they are impossible (or very difficult) to access. The positions of the cross marks (×) correspond to the lower part (row numbers 4.5 to 5, column numbers 1 to 15.5) which holds the proximal end of the leaflet LF inside and the lower part (row numbers 2 and 3 to 4, column number 3) which is located from the commissures CM of the leaflet LF to the former lower part. Note that the cells CL corresponding to the positions of the commissures CM are represented by the addresses (3, 3) (8, 3) and (13, 3) of (column number, row number). The cells CL represented by the addresses (3, 2) (8, 2) and (13, 2) immediately above the addresses (3, 3) (8, 3) and (13, 3) are regions where the leaflet LF near the commissures CM is attached to the frame FR.

The cells CL corresponding to the positions of the inverted triangles (∇) are given low evaluation values because they overlap with the bioprosthetic valve (leaflet LF and skirt SK) and thus have a small clearance and are difficult to access. The positions of the inverted triangles (∇) correspond to the regions adjacent to the lower part and sandwiched between the lower parts of the commissures.

The cells CL corresponding to the positions of the triangles (Δ) can be accessed but is given somewhat low evaluation values because they are close to the junction with the bioprosthetic valve portion. The positions of the triangles (Δ) correspond to the regions adjacent to the lower parts of the commissures except for the upper part and the positions of the inverted triangles (∇) adjacent to the lower part.

The cells CL corresponding to the positions of the circles (o) are given high evaluation values because they can be accessed, but are evaluated somewhat lower than those of the highest evaluation values because they are located lower than the height at which the bioprosthetic valve exists and may interfere with the bioprosthetic valve portion which open and close at the time of access. The positions of the circles (o) correspond to the region sandwiched between the positions of the triangles (Δ) except for the upper part.

The cells CL corresponding to the star marks (☆) are given the highest evaluation values because they have no interference and can easily be accessed. The positions of the star marks (☆) correspond to the upper part with which the leaflet LF that opens and closes does not interfere.

In addition to the above, the evaluation information represents the results of evaluating, among the cells CL, a first blocked portion (×) corresponding to the lower part that holds the leaflet LF of the prosthetic valve VL and a second blocked portion (×) corresponding to the lower part of the commissures located from the commissure portion CM of the leaflets LF to the lower part, relatively lower than the openings (☆, ∘, Δ, ∇) which are different from the first and second blocked portions.

The evaluation information includes a result of evaluating an upper opening (☆) located in the upper part on the opposite side of the lower part among the openings (☆, o, Δ, ∇), relatively higher than intermediate openings (o, Δ, ∇) different from the upper opening.

The evaluation information also includes a result of evaluating a first adjacent opening (V) adjacent to the first blocked portion (×) among the intermediate openings (o, Δ, ∇), relatively lower than first intermediate openings (o, Δ) different from the first adjacent opening.

The evaluation information also includes a result of evaluating a second adjacent opening (Δ) adjacent to the second blocked portion (×) among the first intermediate openings (o, Δ), relatively lower than a second intermediate opening (∘) different from the second adjacent opening.

The evaluation results described above can be acquired for each of the cells CL by a logical evaluation formula as shown in the following (a) to (d).
(a) Determine whether each cell CL is one of the first and second blocked portions or not. If each cell CL is the first or second blocked portion, it is given the lowest evaluation result (×). If not, each cell CL is openings (☆, o, Δ, ∇).
(b) Determine whether each cell CL is an upper opening among the openings (☆, o, Δ, ∇) in the above (a). If each cell CL is an upper opening, it is given the highest evaluation result (☆) . If not, each cell CL is intermediate openings (o, Δ, V).
(c) Determine whether each cell CL is a first adjacent opening among the intermediate openings (o, Δ, ∇). If each cell is a first adjacent opening, it is given the second lowest evaluation result (V). If not, each cell CL is first intermediate openings (o, Δ).
(d) Determine whether each cell CL is a second adjacent opening among the first intermediate openings (o, Δ). If each cell CL is a second adjacent opening, it is given the third lowest evaluation result (Δ). If not, each cell CL is a second intermediate opening (o).

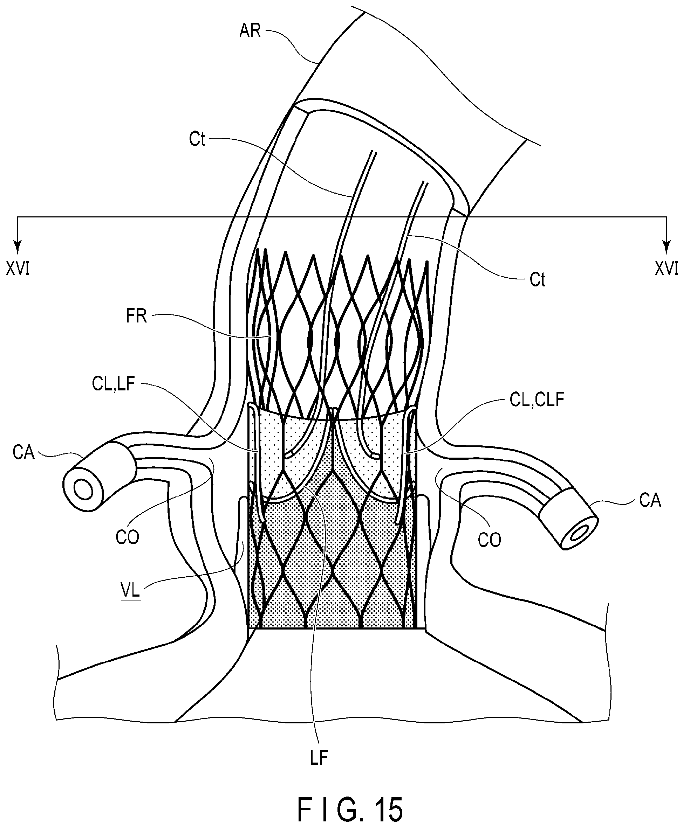

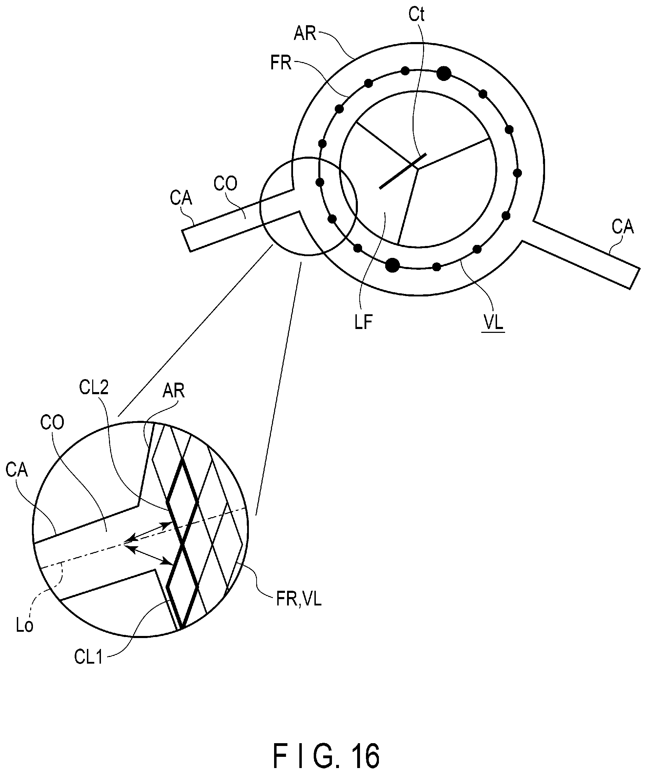

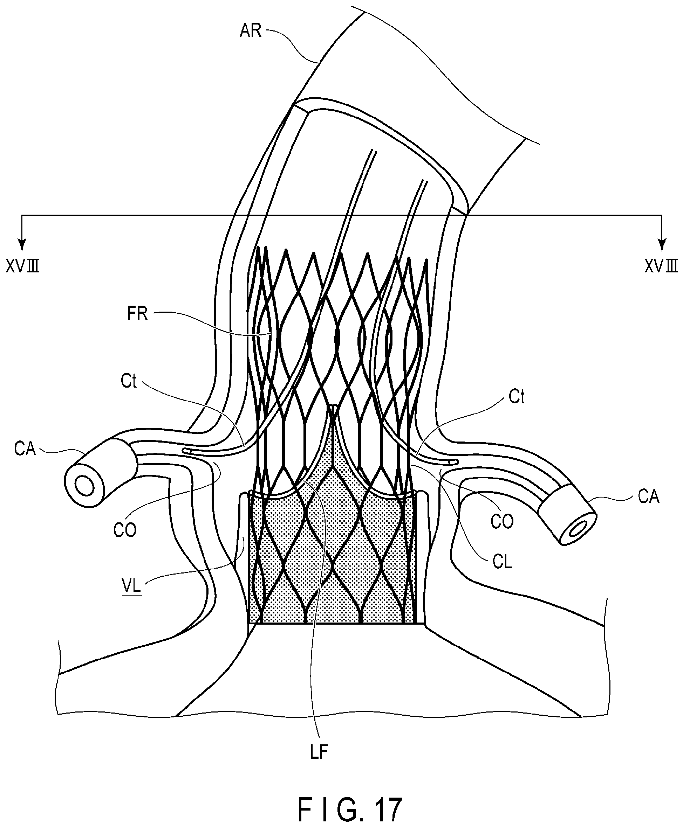

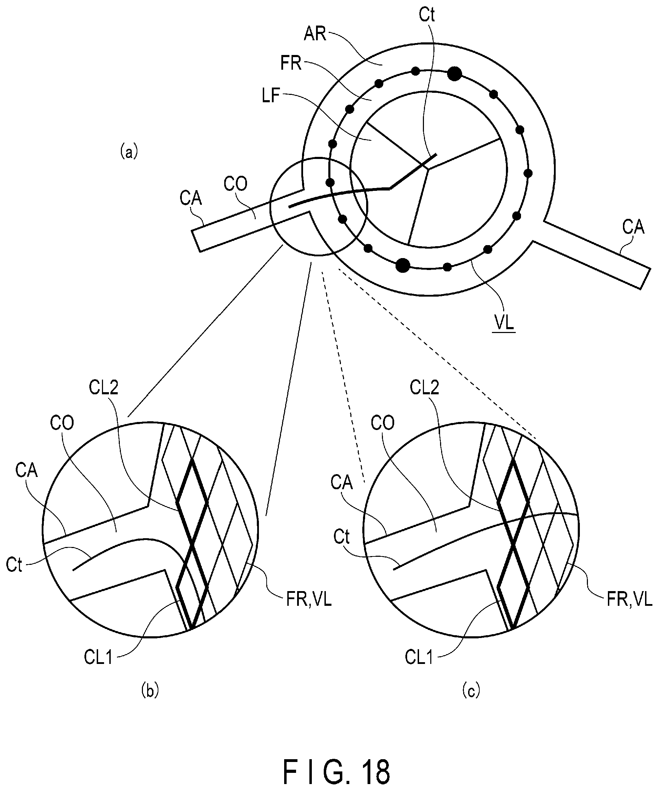

On the other hand, the evaluation values may be calculated based not only on the structure of the prosthetic valve VL but also on the positional relationship between the region of interest of the biological organ and the openings of the prosthetic valve VL. For example, the processing circuitry 134 may calculate the evaluation values based on the distance between the region of interest and each of the openings. In this example, the processing circuitry 134 sets the evaluation values of the cells such that they increase with the decreasing distance from the coronary artery ostium. Alternatively, the processing circuitry 134 may set the evaluation values of the cells located at a certain distance or more from the coronary artery ostium to 0. Alternatively, the processing circuitry 134 may evaluate accessibility to the coronary artery ostium based on the orientation of the coronary artery ostium to correct the evaluation values based on the distance between each of the cells and the coronary artery ostium. In addition, it is assumed that, as shown in FIGS. 15 and 16, a catheter Ct inserted into an aorta AR is to be inserted into a coronary artery ostium CO through one of cells CL1 and CL2 of a prosthetic valve VL. It is assumed here that the cells CL1 and CL2 are located at substantially the same distance from the coronary artery ostium CO. It is also assumed that the cell CL1 is not located but the cell CL2 is located on an extension line of a core line Lo near the coronary artery ostium CO extended in the direction in the aorta AR. In this case, the cell CL2 is easier to access than the cell CL1. For example, as shown in FIGS. 17 and 18(a), if the catheter Ct is inserted into the coronary artery ostium CO through the cell CL1, it needs to be bent greatly as shown in FIG. 18(b). In contrast, if the catheter Ct is inserted through the cell CL2, it needs to be bent more greatly that in the case where the catheter Ct is inserted through the cell CL1, as shown in FIG. 18(c). Thus, it is easier to gain access of the catheter Ct to the coronary artery ostium CO in using the cell CL2 than in using the cell CL1. Therefore, the evaluation values are corrected so that the cell CL2 has a higher evaluation value than the cell CL1.

Note that the processing circuitry 134 may calculate the foregoing evaluation values separately, may calculate each evaluation value based on a predetermined evaluation formula, or may calculate only a single evaluation value selected by the user. The evaluation values calculated as described above are examples and are not particularly limited. That is, the processing circuitry 134 can create evaluation information using an optional evaluation value or evaluation formula.

### (Step S6)

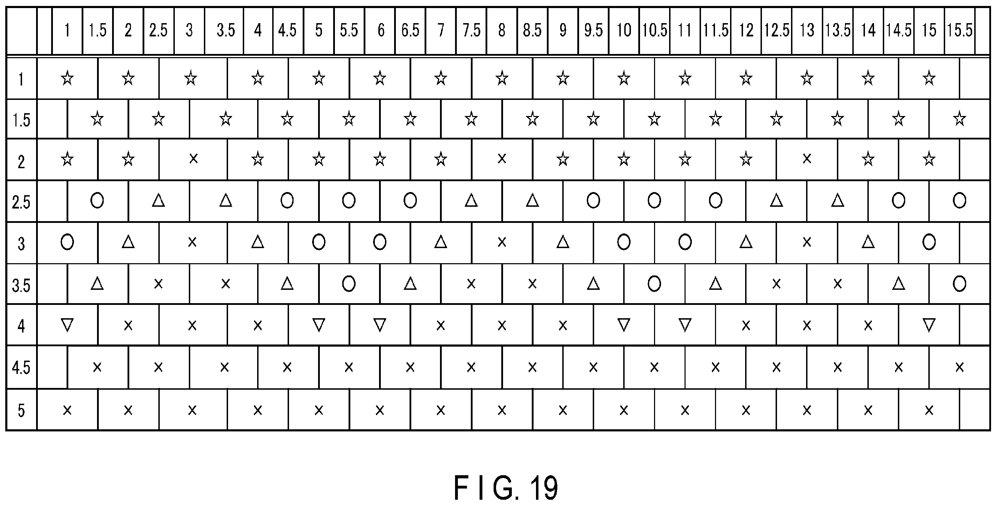

The processing circuitry 134 causes the display control function 134b to display evaluation information on a display 132. Various examples of the evaluation information will be described below with reference to FIGS. 19 to 27.

Like FIG. 14, FIGS. 19 to 21 are diagrams showing various examples of evaluation information representing the evaluation result of each of the cells CL in the prosthetic valve VL shown in FIG. 7. In FIGS. 19 to 21, however, the evaluation information can specify the locations of the cells by the addresses of the row and column numbers. The addresses may be read as circumferential-direction and height-direction positions). As in FIG. 14, the evaluation information defines the location of each cell CL of the prosthetic valve VL with reference to the location of a marker MK1 of the top CR (not shown). That is, the location (1, 1) in the evaluation information of FIGS. 19 to 21 indicates a cell located immediately below the marker MK1 of the prosthetic valve VL shown in FIG. 7. As in FIG. 14, the column number indicates a location in the circumferential direction of the prosthetic valve VL, and the row number indicates a location in the height direction of the prosthetic valve VL. The evaluation information represents the result of evaluating each cell at the defined location.

That is, as shown in FIGS. 19 to 21, the evaluation information is created as a tabular net in which the frame FR is developed at specific locations to define each cell CL as a square, so that the overall image of the evaluation results can easily be recognized in a two-dimensional diagram. The evaluation information is an example of information that represents the evaluation results in a table format in correspondence with the places in the circumferential and height directions.

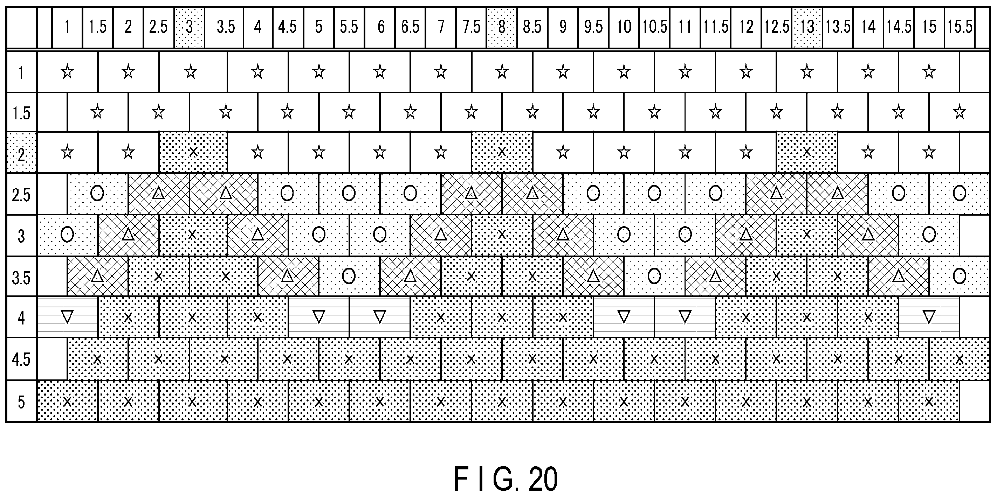

By displaying the above evaluation information on the display 132, the processing circuitry 134 represents the result of evaluation of each cell CL by a display attribute in a square corresponding to the cell CL. For example, the processing circuitry 134 displays the result of evaluation of each cell CL on the display 132 by the evaluation value of the cell CL itself or display attributes such as numerical values, symbols, images, characters, colors and marks which correspond to the evaluation value. Note that in the case of the evaluation information shown in FIGS. 19 to 21, numerical values or symbols corresponding only to the evaluation values calculated based on the form information of a treatment device are displayed. For example, in the case of the evaluation information shown in FIG. 19, a symbol (☆, O, Δ, V, ×) corresponding to the evaluation value is displayed in each square, but the evaluation information is not limited to this display, and numerical information representing the evaluation value may be displayed. In the case of the evaluation information shown in FIG. 20, in addition to the symbols, a color (a pattern in the figure) corresponding to the evaluation value is displayed for each square, but colors may be displayed in place of the symbols. Hereinafter, similarly, the pattern of squares in the figure showing the evaluation information represents the coloring of the squares or the coloring of character strings in the squares.

The processing circuitry 134 may highlight a number indicating a characteristic location in the numerical values indicating the column and row numbers in the evaluation information. In the case of the evaluation information shown in FIG. 20, the column numbers (3, 8, 13) and row number (2) indicating the cells CL corresponding to the locations of the commissures CM of the prosthetic valve VL are colored. In addition to the locations of the commissures CM, the column numbers corresponding to the locations of symbols such as markers MK1 and MK2 may be highlighted, and the column and row numbers corresponding to the location of the coronary artery ostium CO may be highlighted.

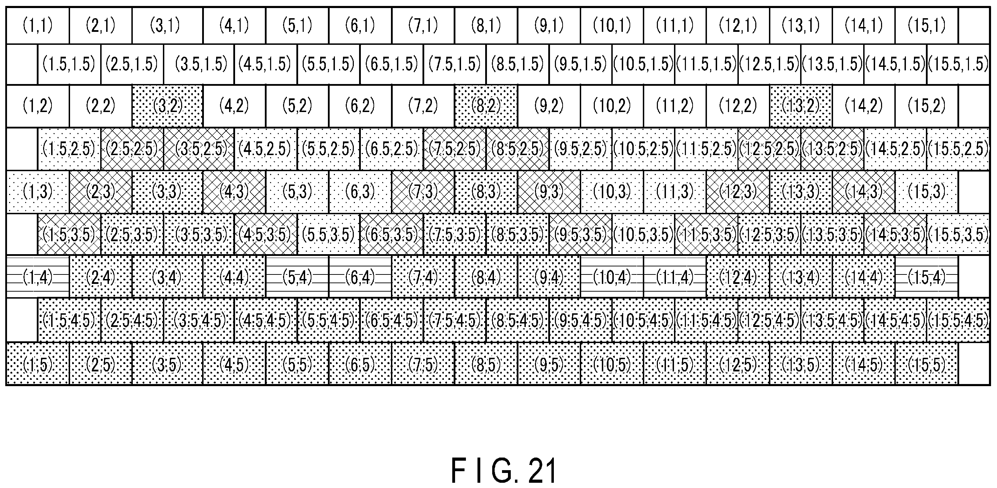

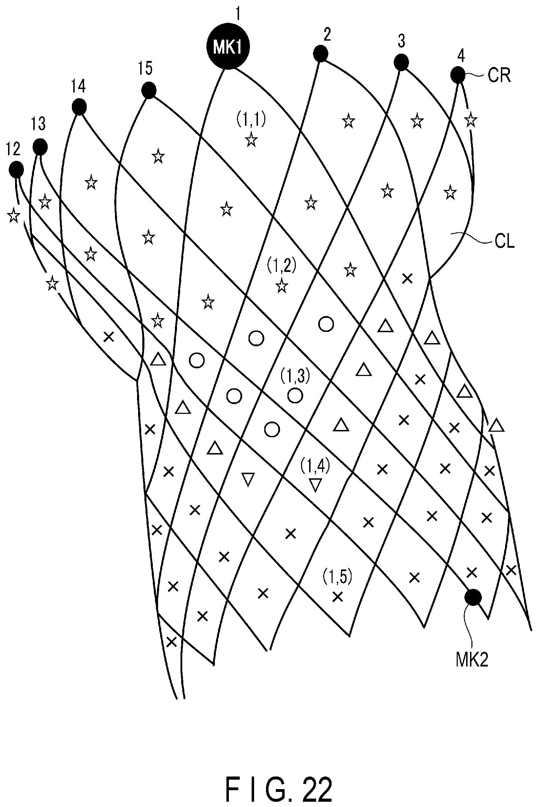

Further, as shown in FIG. 21, the processing circuitry 134 may control the display 132 so as to display an address (column number, row number) in each cell CL based on the evaluation information and to display the color of a character string indicating the address in a color corresponding to the evaluation. Note that in FIG. 21, the color of the character string is expressed by a pattern of squares. These display forms are examples, and any display form can be used as long as the evaluation value in each of the openings can be recognized. For example, as shown in FIG. 22, the processing circuitry 134 may control the display 132 such that each cell in a three-dimensional model corresponding to the form of a treatment device is displayed by a display attribute corresponding to the evaluation value. The evaluation information using the three-dimensional model is an example of information in which the evaluation results are superimposed on the schematic diagram of the frame FR in correspondence with the circumferential and height direction. Note that instead of the three-dimensional model, the picture of the frame FR may be used in place of the three-dimensional model.

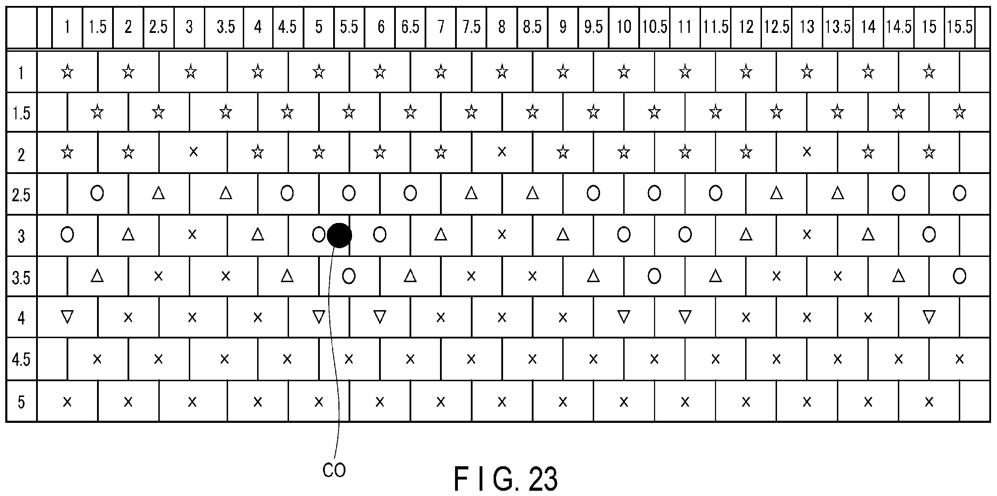

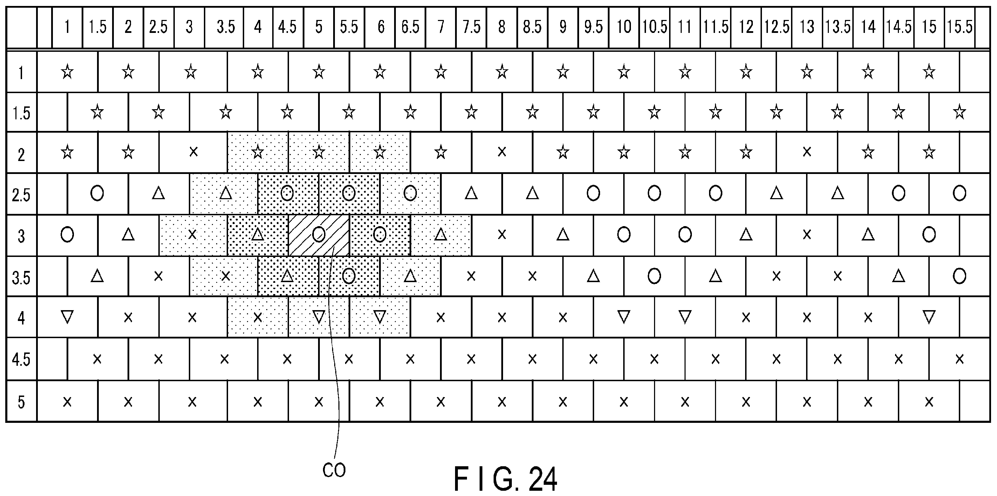

The evaluation information may also include a result of evaluating, among a plurality of openings of a treatment device, an opening located close to a region of interest of a biological organ relatively higher than the other openings. Accordingly, as shown in FIG. 23, the processing circuitry 134 may display a symbol or mark indicating the location of the coronary artery ostium CO which is superimposed on the evaluation information of each cell CL. In FIG. 23, the black circle CO in the address (3, 5) of a cell CL indicates the location of the coronary artery ostium CO. The address (3, 5) where the black circle CO is located is specified as the location of the cell CL closest to the frame FR of the prosthetic valve VL from each location of the coronary artery ostium CO. Thus, the relationship between the coronary artery CA to be treated and each cell CL of the retained prosthetic valve VL can easily be visually recognized. In addition, the display form of each cell CL may be changed in accordance with the evaluation value calculated based on the location of the coronary artery ostium CO. FIG. 24 shows an example of displaying the evaluation information shown in FIG. 19 with the distance from the location of the coronary artery ostium CO as a second evaluation value and with a color (a pattern in the figure) corresponding to the second evaluation value superimposed thereon. The second evaluation value is an evaluation value in which a cell CL located close to the coronary artery ostium CO is evaluated relatively higher than the other cells CL. As shown in FIG. 24, a first evaluation value calculated based on the form information of the treatment device and a second evaluation value calculated based on the positional relationship between a region of interest of a biological organ and the openings of the treatment device may be displayed in different display forms **(e.g.,** symbol and color).

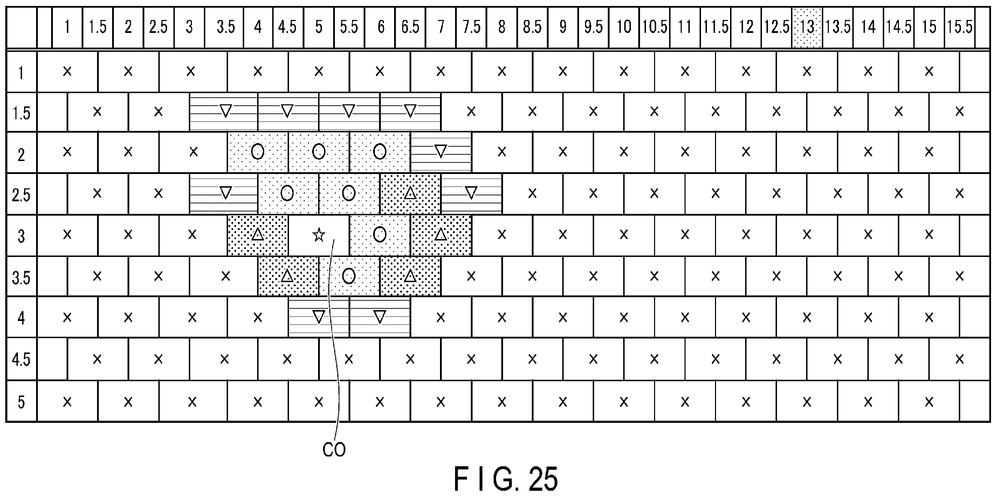

As shown in FIG. 25, the processing circuitry 134 may also control the display 132 to calculate a third evaluation value into which the first and second evaluation values are integrated and to change the display form in accordance with the third evaluation value. Specifically, for example, the highest value (☆) of the second evaluation value may be used as the third evaluation value as it is. At a first adjacent location adjacent to the location of the highest value (☆) of the second evaluation value, the first evaluation value may be used as the third evaluation value as it is. At a second adjacent location adjacent to the first adjacent location and above the location of the highest value, an evaluation value that is made lower than the first evaluation value by one stage may be used as the third evaluation value. At a third adjacent location adjacent to the second adjacent location and above the location of the highest value, an evaluation value that is made lower than the first evaluation value by three stages may be used as the third evaluation value. At all of the other locations, the same evaluation value as those of the blocked portions may be used as the third evaluation value.

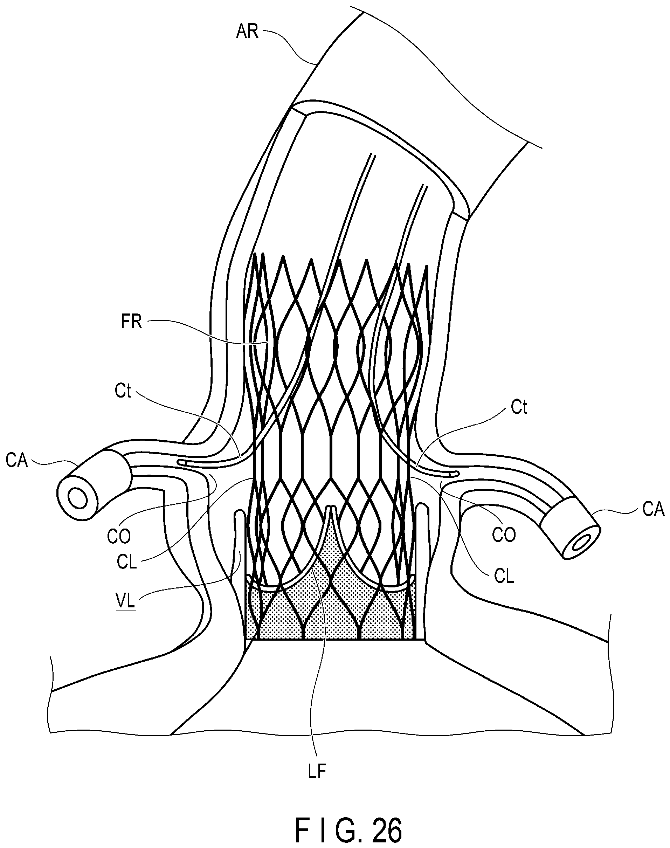

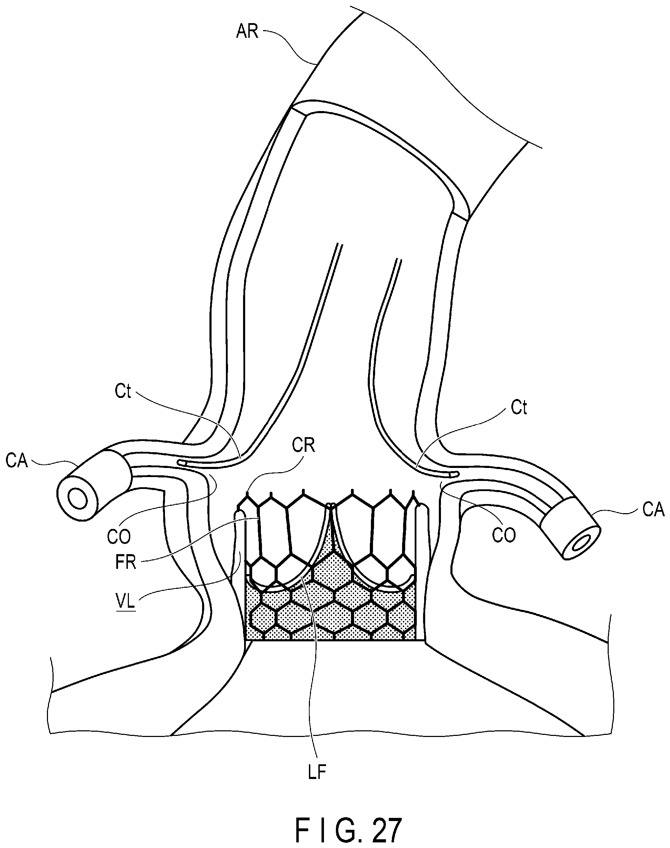

The display form described here is an example, and any display form may be used as long as the evaluation values of the openings can be recognized. If different evaluation values are combined, they may be displayed in a desired form such as a superimposed form of symbols and colors as long as their combinations are recognizable to each of them. Alternatively, display diagrams (geometric nets, three-dimensional models, etc.) which vary in type of evaluation value may be created and displayed side by side, and transmittance may be set for the display diagrams for superimposition display. Alternatively, as shown in FIG. 26, if the location of the leaflet LF in the prosthetic valve VL is lower than that of the coronary artery ostium CO, evaluation information in which all of the evaluation values below the coronary artery ostium CO are set to 0 may be displayed. Alternatively, as shown in FIG. 27, if the location of the top CR of the prosthetic valve VL is lower than that of the coronary artery ostium CO, the display of the evaluation information may be omitted, and a message indicating the omission of the display of the evaluation information may be displayed.

As discussed above, according to the embodiment, the processing circuitry 134 specifies the location of a region of interest of a biological organ from a three-dimensional medical image containing image information regarding a treatment device having a plurality of openings.

The processing circuitry 134 specifies the locations of the openings from the three-dimensional medical image. The processing circuitry 134 evaluates at least one of the openings based on the location of the region of interest and the locations of the openings. Thus, the positional relationship between a region of interest of a biological organ of each patient and each of the openings of a treatment device can easily be grasped by evaluating the openings of the treatment device from three-dimensional medical images of the patient. In addition, more appropriate medical diagnosis and treatment can be performed, for example, by analyzing medical images to obtain the locations of a biological organ and a treatment device of a patient.

In addition, according to the embodiment, the processing circuitry 134 may create evaluation information representing the results of evaluation in association with the locations of the openings and display the evaluation information on the display 132. In this case, in addition to the advantages described above, the user can visually recognize the positional relationship between a region of interest of the biological organ and each of the openings of the treatment device. Also, for example, the evaluation values of the openings in the treatment device can be displayed in association with relative locations of the openings.

According to the embodiment, the treatment device may be a prosthetic valve VL including a mesh-like substantially cylindrical frame FR including a plurality of cells CL. The openings may be a plurality of cells CL. In this case, in addition to the advantages described above, when a patient in which the prosthetic valve VL is retained is treated, the positional relationship between a region of interest of the biological organ and each cell CL of the frame FR of the prosthetic valve VL can easily be grasped. Furthermore, based on the positional relationship between the prosthetic valve VL and the coronary artery ostium CO, the position of a hole (cell) of the prosthetic valve VL to be targeted in the postoperative PCI can be specified. Thus, the postoperative PCI can be performed more appropriately to bring about advantages such as improvement of treatment success rate and reduction of operation time.

In addition, according to the embodiment, the positions of the cells CL may be indices corresponding to the places of the substantially cylindrical frame in the circumferential direction and the places thereof in the height direction. In this case, in addition to the advantages described above, the positions of the cells CL can be indicated by the places in the circumferential and height directions.

In addition, according to the embodiment, the evaluation information may be either information in which the evaluation results are represented in a tabular format in association with the places in the circumferential and height directions or information in which the evaluation results are superimposed on the schematic diagram or picture of the frame FR and represented in association with the places in the circumferential and height directions. In this case, in addition to the advantages described above, the evaluation information can be displayed in a format such as a table, a schematic diagram and a picture.

In addition, according to the embodiment, the evaluation information may be information indicating the evaluation results by display attributes corresponding to the evaluation results. In this case, in addition to the advantages described above, the user can intuitively grasp the evaluation results in accordance with the display attributes.

In addition, according to the embodiment, the evaluation information may include a result of evaluating one of the openings, which is located close to a region of interest, relatively higher than the other openings. In this case, in addition to the advantages described above, the advantage that a catheter reaches a region of interest simply by inserting the catheter through the highly evaluated opening.

According to the embodiment, the evaluation information may represent a result of evaluating, among a plurality of openings, a first blocked portion (×) corresponding to a lower part located inside the heart valve of a subject and a second blocked portion (×) located from the commissures CM of the leaflet LF of the prosthetic valve VL to the lower part, relatively lower than the openings (☆, o, Δ, ∇) different from the first and second blocked portions. In this case, in addition to the advantages described above, the evaluation of the first and second blocked portions (×) can be made the lowest.

In addition, according to the embodiment, the evaluation information may include a result of evaluating, among the openings (☆, o, Δ, ∇) different from the first and second blocked portions, an upper opening (☆) located at an upper part on the opposite side of the lower part, relatively higher than intermediate openings (o, Δ, ∇) different from the upper opening. In this case, in addition to the advantages described above, the evaluation of the upper opening (☆) can be made the highest.

According to the embodiment, the evaluation information may include a result of evaluating, among the intermediate openings (o, Δ, V), a first adjacent opening (V) adjacent to the first blocked portion (×), relatively lower than first intermediate openings (o, Δ) different from the first adjacent opening. In this case, in addition to the advantages described above, the evaluation of the first adjacent opening (V) can be made low in the intermediate openings (o, Δ, V).

According to the embodiment, the evaluation information may include a result of evaluating, among the first intermediate openings (o, Δ), a second adjacent opening (Δ) adjacent to the second blocked portion (×), relatively lower than the second intermediate opening (∘) different from the second adjacent opening. In this case, in addition to the advantages described above, the evaluation of the second adjacent opening (Δ) can be made low in the first intermediate openings (o, Δ).

In addition, according to the embodiment, the processing circuitry 134 may acquire form information of a treatment device. The processing circuitry 134 may evaluate at least one of a plurality of openings based on the form information, the position of a region of interest, and the positions of the openings. In this case, in addition to the advantages described above, an evaluation result can be obtained based on the form information of the treatment device.

### (First Modification)

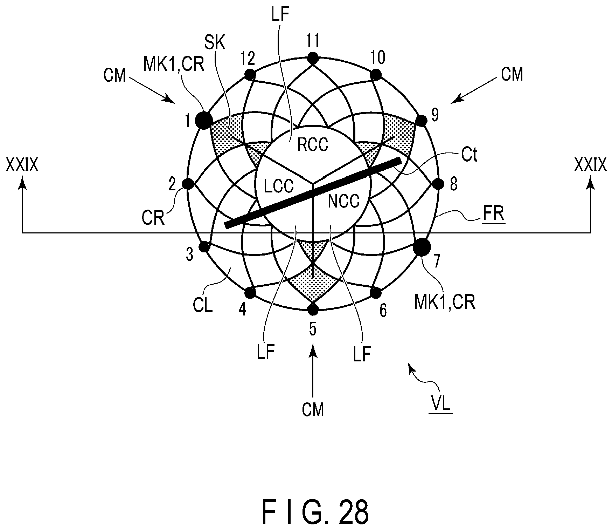

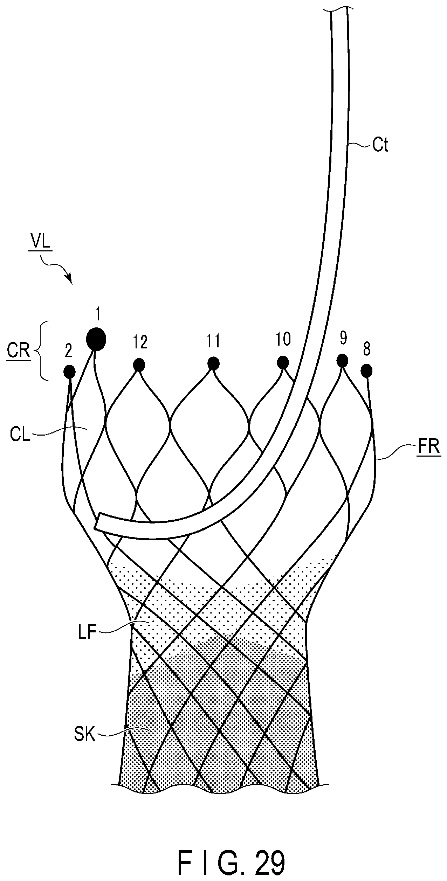

In the foregoing embodiment, an evaluation value is calculated based on the form information of a treatment device and the positional relationship between a region of interest of a biological organ and the openings of the treatment device, but this is not a limitation. For example, the evaluation value may be calculated or corrected based on the type of a catheter used for PCI after TAVI in addition to the form information and the positional relationship. For example, the processing circuitry 134 may cause the acquisition function 134a to acquire the shape information of a catheter for use in the treatment of a patient. The acquisition function 134a is an example of the second acquisition unit. For example, the processing circuitry 134 may cause the evaluation function 134d to evaluate at least one of a plurality of openings based on the shape information of the catheter, the position of a region of interest, and the positions of the openings. The shape information of the catheter is information for specifying the type and shape of the catheter. As the type of a catheter, for example, Judkins left (JL), Judkins right (JR), Amplatz left (AL) and Amplatz right (AR) can be used as appropriate. As the shape of the catheter, for example, the position of a bend portion and the radius of curvature of the bent portion can be used as appropriate. That is, the shape information of the catheter is information that can specify the shape of the catheter based on the type of the catheter and the shapes of various bent portions. In addition, as shown in FIGS. 28 and 29, the shape of a catheter Ct needs to be specified because the optimal cell CL for accessing the ostium of a coronary artery varies particularly with the shape of the distal end of the catheter Ct.

Specifically, for example, in step S1, the processing circuitry 134 may acquire shape information of a catheter that is expected to be used in PCI after TAVI. In step S5, the processing circuitry 134 may evaluate the accessibility of the catheter Ct to each cell CL based on the shape information of the catheter, the position of the coronary artery ostium CO and the position of each cell CL, and calculate an evaluation value based on a result of the evaluation.

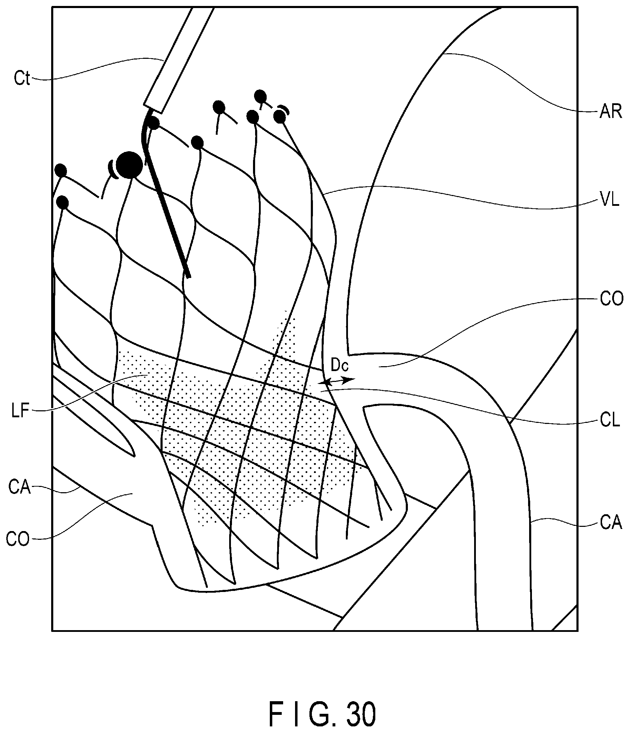

The processing circuitry 134 is not limited to the above evaluation or calculation, but may calculate or correct an evaluation value based on the distance Dc between the cell CL into which the catheter Ct inserted and the coronary artery ostium CO, as shown in FIG. 30.

According to the first modification as described above, the processing circuitry 134 acquires the shape information of a catheter for use in the treatment of a patient. The processing circuitry 134 evaluates at least one of a plurality of openings based on the shape information of the catheter, the position of a region of interest, and the positions of the openings. It is thus possible to obtain the evaluation result in consideration of the shape of the catheter in addition to the advantages of the embodiment.

### (Second Modification)

In the embodiment and the first modification, evaluation information is displayed at the time of treatment planning, but this is not a limitation. For example, in an intraoperative fluoroscopic X-ray image, the position of a cell corresponding to an evaluation result may be highlighted. Note that a medical image processing apparatus 130 according to a second modification is preferably mounted on a medical image diagnostic apparatus 110 such as an X-ray diagnostic apparatus. However, this is not a limitation. The medical image processing apparatus 130 may specify the position of each cell CL of a prosthetic valve VL from a fluoroscopic X-ray image based on the known technology, and display the cell CL in a display form corresponding to an evaluation value. In this case, an evaluation value need not be set to all cells CL, but a cell to be superimposed and displayed may be specified based on an evaluation value, and only the position of the cell may be specified from a fluoroscopic X-ray image and highlighted to correspond to the evaluation value. Note that as the cell CL to be superimposed and displayed, for example, a predetermined number of cells CL may be selected in order of increasing evaluation values. Also, as a method for specifying each cell of the treatment device from the fluoroscopic X-ray image, for example, the known technologies described in References 1 and 2 below may be used.

Reference 1: Gopalakrishnan, Vivek and Polina Golland, "Fast Auto-Differentiable Digitally Reconstructed Radiographs for Solving Inverse Problems in Intraoperative Imaging," Workshop on Clinical Image-Based Procedures, Cham: Springer Nature Switzerland, 2022

Reference 2: Unberath, Mathias et al., "DeepDRR-a Catalyst for Machine Learning in Fluoroscopy-Guided Procedures," Medical Image Computing and Computer Assisted Intervention-MICCAI 2018: 21st International Conference, Granada, Spain, September 16-20, 2018, Proceedings, Part IV 11, Springer International Publishing, 2018

In this case, each cell can be specified by generating a virtual fluoroscopic X-ray image from CT images of a patient and registering the generated virtual fluoroscopic X-ray image and the actual intraoperative fluoroscopic X-ray image. As the alignment method, a known linear or nonlinear deformation registration method can be used. Specifically, for example, a known deformation registration method such as a free-form deformation (FFD) method and a large deformation differential metric mapping (LDDMM) method can be used as appropriate.

Accordingly, the processing circuitry 134 causes the acquisition function 134a to acquire an X-ray image including a treatment device of a patient. The acquisition function 134a is an example of the third acquisition unit. The processing circuitry 134 causes the display control function 134b to display the X-ray image on the display 132.

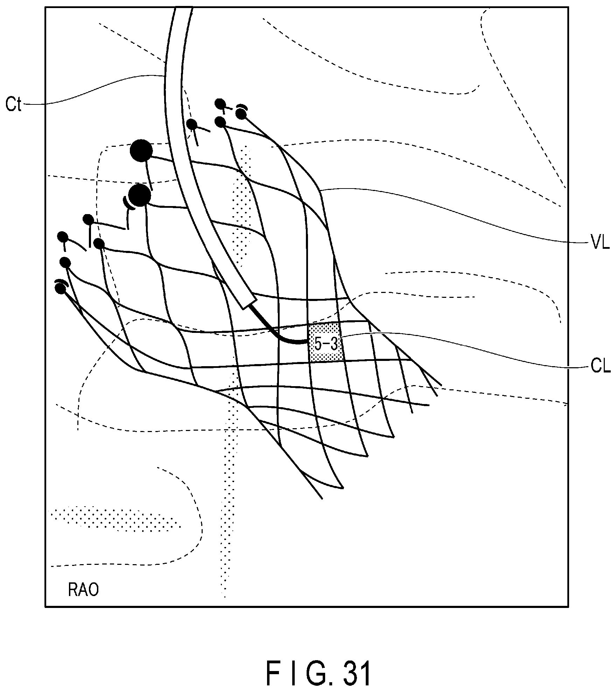

In addition, the processing circuitry 134 selects the position of at least one of the evaluated openings based on the result evaluated in step S5 by the evaluation function 134d. The processing circuitry 134 causes the display control function 134b to highlight on the display 132 a position corresponding to the selected position on the **X-**ray image. For example, as shown in FIG. 31, the processing circuitry 134 causes it on the display 132 to highlight a position corresponding to the selected position (5-3) on the X-ray image.

According to the second modification described above, the processing circuitry 134 acquires an X-ray image including the treatment device of a patient. The processing circuitry 134 displays the X-ray image on the display. The processing circuitry 134 selects the position of at least one of the evaluated openings based on the evaluated result. The processing circuitry 134 highlights a position corresponding to the selected position on the X-ray image on the display 132. Thus, the user ca visually recognize the position of an opening of the treatment device to gain access to a region of interest of the biological organ during operation.

According to at least one embodiment and the modifications described above, the positional relationship between a region of interest of a biological organ of each patient and each of the openings of a treatment device can easily be grasped.

The term "processor" in the above descriptions means, for example, a CPU, a GPU, or a circuit such as an application specific integrated circuits (ASIC), a programmable logic device (e.g., simple programmable logic devices (SPLD)), a complex programmable logic device (CPLD) and a field programmable gate array (FPGA). If the processor is, for example, a CPU, it fulfills a function by reading a program from a memory and executing the program. If the processor is, for example, an ASIC, instead of storing a program in the memory, the function is directly incorporated into a circuit of the processor as a logic circuit. Note that each processor of the embodiment is not limited to the case where the processor is configured as a single circuit, but may be configured as a single processor by combining a plurality of independent circuits to fulfill the function. In addition, a plurality of components shown in FIG. 1 or 32 may be integrated into a single processor to fulfill the function.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the scope of the invention as defined by the appended claims.

## Claims

1. A medical image processing apparatus comprising:
a first specifying unit (134c) configured to specify a position of a region of interest of a biological organ from a three-dimensional medical image including image information regarding a treatment device having a plurality of openings;
a second specifying unit (134c) configured to specify positions of the openings from the three-dimensional medical image; and
an evaluation unit (134d) configured to evaluate at least one of the openings based on the position of the region of interest and positions of the openings.

2. The medical image processing apparatus according to claim 1, further comprising a display control unit (134b),
wherein the evaluation unit (134d) is configured to create evaluation information indicating a result of the evaluation in association with the positions of the openings; and
the display control unit (134b) is configured to display the evaluation information on a display (132).

3. The medical image processing apparatus according to claim 2, wherein the treatment device is a prosthetic valve (VL) including a mesh-like substantially cylindrical frame (FR) including a plurality of cells (CL); and the openings correspond to the cells (CL).

4. The medical image processing apparatus according to claim 3, wherein positions of the cells (CL) are indices corresponding to places of the substantially cylindrical frame (FR) in a circumferential direction and places thereof in a height direction.

5. The medical image processing apparatus according to claim 4, wherein the evaluation information is one of information representing the result of the evaluation in a tabular format in association with the places in the circumferential direction and the places in the height direction and information representing the result of the evaluation, which is superimposed on one of a schematic diagram and a picture of the frame (FR), in association with the places in the circumferential direction and the places in the height direction.

6. The medical image processing apparatus according to any one of claims 2 to 5, wherein the evaluation information is information representing the result of the evaluation by a display attribute corresponding to the result of the evaluation.

7. The medical image processing apparatus according to any one of claims 2 to 5, wherein the evaluation information includes a result of evaluating, among the openings, an opening located close to the region of interest relatively higher than other openings.

8. The medical image processing apparatus according to any one of claims 3 to 5, wherein the evaluation information represents a result of evaluating, among the openings, a first blocked portion (x) corresponding to a lower part located inside a heart valve of a subject and a second blocked portion located from a commissure of leaflets (LF) of the prosthetic valve (VL) to the lower part relatively lower than openings different from the first blocked portion (x) and the second blocked portion (x).

9. The medical image processing apparatus according to claim 8, wherein the evaluation information includes a result of evaluating, among the different openings, an upper opening located at an upper part on an opposite side of the lower part relatively higher than intermediate openings different from the upper opening.

10. The medical image processing apparatus according to claim 9, wherein the evaluation information includes a result of evaluating, among the intermediate openings, a first adjacent opening adjacent to the first blocked portion (x) relatively lower than first intermediate openings different from the first adjacent opening.

11. The medical image processing apparatus according to claim 10, wherein the evaluation information includes a result of evaluating, among the first intermediate openings, a second adjacent opening adjacent to the second blocked portion (x) relatively lower than a second intermediate opening different from the second adjacent opening.

12. The medical image processing apparatus according to claim 1, further comprising a first acquisition unit (134a) configured to acquire form information of the treatment device,
wherein the evaluation unit (134d) is configured to evaluate at least one of the openings based on the form information, the position of the region of interest, and the positions of the openings.

13. The medical image processing apparatus according to claim 1, further comprising a second acquisition unit (134a) configured to acquire shape information of a catheter (CT) for use in treatment of a subject,
wherein the evaluation unit (134d) is configured to evaluate at least one of the openings based on the shape information, the position of the region of interest, and the positions of the openings.

14. The medical image processing apparatus according to claim 1, further comprising:
a third acquisition unit (134a) configured to acquire an X-ray image including the treatment device; and
a display control unit (134b) configured to display the X-ray image on a display,
wherein the evaluation unit (134d) is configured to select a position of the evaluated at least one of the openings based on a result of the evaluation; and
the display control unit (134b) is configured to highlight on the display a position corresponding to the selected position on the X-ray image.

15. A medical image processing method comprising:
specifying a position of a region of interest of a biological organ from a three-dimensional medical image including image information regarding a treatment device having a plurality of openings;
specifying positions of the openings from the three-dimensional medical image; and
evaluating at least one of the openings based on the position of the region of interest and positions of the openings.

16. A program including computer executable instructions, wherein the instructions, when executed by a processor, cause the processor to perform a method comprising:
specifying a position of a region of interest of a biological organ from a three-dimensional medical image including image information regarding a treatment device having a plurality of openings;
specifying positions of the openings from the three-dimensional medical image; and
evaluating at least one of the openings based on the position of the region of interest and positions of the openings.
